(19) Europäisches Patentamt
European Patent Office
Office européen des brevets

(11) **EP 4 741 505 A1**

(12) **EUROPEAN PATENT APPLICATION**
published in accordance with Art. 153(4) EPC

(43) Date of publication:
**13.05.2026 Bulletin 2026/20**

(21) Application number: **25773114.1**

(22) Date of filing: **21.03.2025**

(51) International Patent Classification (IPC):
*C12N 15/113* (2010.01)      *C12P 13/00* (2006.01)
*C12R 1/225* (2006.01)       *C07C 229/22* (2006.01)
*C12R 1/19* (2006.01)        *C12P 21/02* (2006.01)
*C12N 15/74* (2006.01)

(86) International application number:
**PCT/CN2025/084237**

(87) International publication number:
**WO 2025/195516 (25.09.2025 Gazette 2025/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.03.2024   CN 202410335441**

(71) Applicants:
• **Innobio Corporation Limited**
  **Dalian, Liaoning 116600 (CN)**
• **Innobio (Hei Long Jiang) Limited**
  **Jiamusi, Heilongjiang 156100 (CN)**

(72) Inventors:
• **FAN, Chao**
  **Dalian, Liaoning 116600 (CN)**
• **LIU, Jun**
  **Dalian, Liaoning 116600 (CN)**

• **QI, Jiakun**
  **Dalian, Liaoning 116600 (CN)**
• **CAI, Zhaoning**
  **Dalian, Liaoning 116600 (CN)**
• **HONG, Hao**
  **Dalian, Liaoning 116600 (CN)**
• **CHEN, Jianbin**
  **Dalian, Liaoning 116600 (CN)**
• **WU, Wenzhong**
  **Dalian, Liaoning 116600 (CN)**

(74) Representative: **Dai, Simin**
  **Reyda IP**
  **A073**
  **157, Quai du Président Roosevelt**
  **92130 Issy-les-Moulineaux (FR)**

Remarks:
The complete document including Reference
Table(s) and the Sequence Listing(s) can be
downloaded from the EPO website

(54) **NON-CODING SRNA IN CORYNEBACTERIUM GLUTAMICUM AND USE THEREOF**

(57)    Provided is a non-coding small RNA (sRNA) from *Corynebacterium glutamicum* and use thereof. An RNA sequence of the non-coding sRNA has at least 90% or more sequence identity to a transcription product of a DNA sequence shown in SEQ ID NO.1. Experimental results demonstrate that overexpression of the non-coding sRNA in the recombinant bacterium can significantly increase yields of the branched-chain amino acids such as L-leucine, L-isoleucine, and L-valine and the sugar-to-acid conversion rate, while reducing generation of the byproduct acids. Regarding raw materials, relatively low-cost molasses is selected as a carbon and nitrogen source, corn steep liquor with a high impurity content is discarded, and molasses is converted via enzymatic hydrolysis into a nutrient containing more carbon and nitrogen sources utilizable by strains. In addition, to control a carbon-to-nitrogen ratio in a fermentation broth, a more precise feeding process is selected, which satisfies fermentation requirements of strains and effectively avoids excessively high or low nitrogen content in the fermentation broth, thereby achieving goals of increasing yield and sugar-to-acid conversion rate and reducing the byproduct acid production.

EP 4 741 505 A1

**Fig 1**

**Description**

**CROSS-REFERENCE TO RELATED APPLICATIONS**

This application claims priority of the Chinese application filed on March 22, 2024, with the application number 202410335441.0, the entire contents of which are incorporated herein by reference.

**TECHNICAL FIELD**

The present disclosure relates to the field of genetic engineering technologies, and in particular to a non-coding small RNA (sRNA) from *Corynebacterium glutamicum* and its use.

**BACKGROUND**

Branched-chain amino acids (c) possess a variety of physiological functions and have very broad applications in industries such as food, animal feed, cosmetics, and medicine. In the early stage, BCAAs were mainly produced by chemical synthesis; however, the chemical synthesis had a low yield and caused severe environmental pollution, thus being gradually eliminated. Subsequently, microbial fermentation attracted widespread attention. At present, strains used for producing the BCAAs mainly include *Brevibacterium flavum, Escherichia coli,* and *Corynebacterium glutamicum.* The biosynthetic pathway of the BCAAs is lengthy and has a complex and precise self-adjustment mechanism. In natural strains, the biosynthetic pathway of amino acids is strictly controlled by metabolism to ensure that amino acids are produced only for intrinsic needs of cells. Therefore, production of the BCAAs through strain modification has attracted increasing attention. With technological advancements, metabolic modification is gradually becoming a main manner for obtaining high-yield amino acid strains. However, the industrial stability of the strains subjected to extensive metabolic modification is not ideal. Although metabolic engineering modification can improve the yield of the amino acids to some extent, as primary metabolites, on one hand, weakening of a synthesis pathway of by-products and metabolic burden caused by adding synthesis modules weakens growth of the strains; on the other hand, key enzymes on the synthesis pathway are mostly subjected to feedback inhibition from intermediate metabolites and final products. A balance between sufficient precursors and alleviation of the feedback inhibition cannot be stably achieved in industrial production. In summary, due to a lack of in-depth understanding and application of fine adjustable mechanisms of microorganisms, limitations exist in optimizing performance of the strains, enhancing stability, and further increasing yield of target products.
Bacterial non-coding small RNAs (sRNAs) are a class of RNA regulators discovered in prokaryotes such as bacteria in recent years. The non-coding sRNAs do not encode proteins, have a length of 50-500 nucleotides, and are extensively involved in regulation of a plurality of life activities in vivo. Bacterial sRNAs are important regulatory factors of bacterial metabolism, virulence, and adaptation to environmental stress, and play an important role in gene expression adjustment in response to environmental changes. However, an action mechanism of non-coding sRNAs in regulating the amino acid biosynthetic pathway is still unclear, and technologies for targeted design and utilization of sRNAs for high-efficiency and precise regulation are lacking.
Therefore, it is necessary to provide a non-coding sRNA from *Corynebacterium glutamicum* and its use, so as to efficiently, low-costly, and continuously separate and purify fermented BCAAs, increasing product purity, reducing impurity content, and achieving recycling of key raw materials.

**SUMMARY**

One or more embodiments of the present disclosure provide a non-coding small RNA (sRNA) from *Corynebacterium glutamicum.* An RNA sequence of the non-coding sRNA has at least 90% sequence identity to a transcription product of a DNA sequence set forth in SEQ ID NO: 1.
In some embodiments, a DNA sequence encoding the non-coding sRNA is set forth in SEQ ID NO:1.
One or more embodiments of the present disclosure provide a vector comprising the DNA sequence encoding the non-coding sRNA.
One or more embodiments of the present disclosure provide a recombinant bacterium expressing the DNA sequence encoding the non-coding sRNA, wherein a bacterial strain of the recombinant bacterium is *Corynebacterium glutamicum.*
In some embodiments, the *Corynebacterium glutamicum* is selected from *Corynebacterium glutamicum* IBBH-15, *Corynebacterium glutamicum* IBCL-1, *Corynebacterium glutamicum* IBCVQ, *Corynebacterium glutamicum* CICC21756, *Corynebacterium glutamicum* ATCC13002, or any combination thereof.
One or more embodiments of the present disclosure provide a method for constructing a recombinant bacterium. The method comprises the following step. A recombinant strain is obtained by transferring a recombinant vector comprising a target DNA fragment into a host strain, wherein the target DNA fragment has at least 90% sequence identity to a DNA sequence set forth in SEQ ID NO: 1.

In some embodiments, a DNA sequence of the target DNA fragment is set forth in SEQ ID NO: 1.

One or more embodiments of the present disclosure provide a fermentation method for a recombinant bacterium. The method comprises the following steps. A seed culture is obtained by inoculating at least one recombinant strain into a seed medium for cultivation. The seed culture is inoculated into a fermentation medium for fermentation cultivation. In the fermentation cultivation, production of branched-chain amino acids is increased by adjusting at least one of an added amount of inducer, a dissolved oxygen level, pH, and nutrient supply.

In some embodiments, the at least one recombinant strain comprises a DNA sequence encoding a non-coding sRNA, and the DNA sequence has at least 90% sequence identity to a DNA sequence set forth in SEQ ID NO:1.

In some embodiments, the recombinant bacterium is an L-leucine-producing strain, the L-leucine-producing strain including at least one of *Corynebacterium glutamicum* mutant strains IBBH-15, IBCLQ-257, and IBCLQ-257e.

In some embodiments, the method further comprises the following steps. A dissolved oxygen level of fermentation cultivation is controlled to be 10-20% during a first 22-26 h of the fermentation cultivation, and the dissolved oxygen level is controlled to be 5-15% after the fermentation cultivation for 22-26 h. An initial pH is controlled to be 6.65-6.75. The pH is increased to 6.85-6.95 in response to a cell OD562 increasing to 19-21. The pH is increased to 6.95-7.05 in response to the cell OD562 increasing to 24-26. The pH is increased to 7.15-7.25 in response to the cell OD562 increasing to 35-37. A residual sugar concentration is controlled to be 20-30 g/L during the fermentation process.

In some embodiments, the seed medium comprises glucose at an initial concentration of 2.5-3.5 wt% and a molasses hydrolysate at an initial concentration of 1-2 wt%, the fermentation medium comprises glucose at an initial concentration of 3.5-4.5 wt% and a molasses hydrolysate at a concentration of 0.5-1.1 wt%, and the molasses hydrolysate is selected from at least one of a beet molasses hydrolysate and a cane molasses hydrolysate.

In some embodiments, a dissolved oxygen level in the fermentation cultivation is 5-10%. The method further comprises the following steps. In the fermentation cultivation, after a cell OD562 is increased to 19-21, the pH is increased to 6.85-6.95, and when a residual sugar concentration is decreased to 1.5-2.5 wt%, a glucose solution having a concentration of 40-50 wt% and a molasses hydrolysate having a concentration of 40-50 wt% are started to be fed. A flow rate ratio of the glucose solution to the molasses hydrolysate is controlled to be 5:1-10:1, and a ratio of the molasses hydrolysate to the fermentation medium is controlled to be 2.5-3.5 wt%. After the cell OD562 is increased to 29-31, the pH is increased to 7.05-7.15. After the cell OD562 is increased to 39-41, the pH is increased to 7.15-7.25, and the residual sugar concentration is controlled to be 1.5-2.5 wt% during the fermentation process.

In some embodiments, the recombinant bacterium is an L-isoleucine-producing strain, the L-isoleucine-producing strain comprising at least one of *Corynebacterium glutamicum* mutant strains IBCIL-253, IBCL-1, and IBCIL-253k.

In some embodiments, the method further comprises the following steps. In response to a residual sugar concentration being lower than 0.8-1.2 wt%, a supplementary sugar solution is fed to control the residual sugar concentration of a fermentation broth to be 0.8-1.2 wt%, and an inorganic salt supplementary solution is simultaneously continued to be fed to control a fermentation broth osmolality to be 890-910 mosm/L.

In some embodiments, the seed medium comprises glucose at an initial concentration of 4.5-5.5 wt% and a molasses hydrolysate at an initial concentration of 2.5-3.5 wt%, the fermentation medium comprises glucose at an initial concentration of 9.5-10.5 wt% and a molasses hydrolysate at a concentration of 1.5-2.5 wt%, and the molasses hydrolysate is selected from at least one of a beet molasses hydrolysate and a cane molasses hydrolysate.

In some embodiments, a dissolved oxygen level in the fermentation cultivation is 8-12%. The method further comprises the following steps. In the fermentation cultivation, after a cell OD562 is increased to 39-41, the pH is increased to 6.85-6.95. When a residual sugar concentration is decreased to 5.5-6.5 wt%, an inorganic salt supplementary solution is fed to control a fermentation broth osmolality to be 690-710 mosm/L. When a residual sugar concentration is decreased to 1.5-2.5 wt%, a supplementary solution A and a supplementary solution B are started to be fed. The supplementary solution A comprises a glucose solution at a concentration of 40-50 wt%, a potassium dihydrogen phosphate solution at a concentration of 0.1 wt%, and a magnesium sulfate solution at a concentration of 0.05 wt%. The supplementary solution B comprises a molasses hydrolysate at a concentration of 40-50 wt%. A flow rate ratio of the supplementary solution A to the supplementary solution B is controlled to be 4:1-7:1. A ratio of the molasses hydrolysate to the fermentation medium is controlled to be 3.5-4.5 wt%. The inorganic salt supplementary solution is continuously fed to control the fermentation broth osmolality to be 890-910 mosm/L.

One or more embodiments of the present disclosure provide a separation and purification method for a fermentation product. The separation and purification method comprises the following steps. The fermentation product is redissolved using p-toluenesulfonic acid, wherein a molar ratio of the fermentation product to the p-toluenesulfonic acid is 1:1-1:2. A hydrogen bond network reconstruction is performed on the redissolved fermentation product using 0.5-1.5 M inorganic acid. The fermentation product is decolorized after cooling crystallization for 30-50 min using 1-2% malic acid, 0.1-0.2% tartaric acid, and 0.5-1.5‰ activated carbon. The decolorized fermentation product is purified by ion exchange resin and evaporation crystallization to obtain a purified fermentation product.

In some embodiments, after ion exchange resin and before evaporation crystallization, the method further comprises the following step.

The p-toluenesulfonic acid is eluted and recovered using 0.3-0.8 M hydrochloric acid, and the resin is regenerated using 2.5-3.5 M hydrochloric acid.

In some embodiments, after the hydrogen bond network reconstruction and before decolorization, the method further comprises the following step. The fermentation product after the hydrogen bond network reconstruction is cooled to 9-11°C at a cooling rate of 8-12°C/h, and seed crystallization is performed for 0.5-1.5 h.

In some embodiments, before redissolution, the method further comprises the following step. A complex precipitation is performed on a fermentation broth containing the fermentation product using a composition of polypeptides and metal ions to precipitate the fermentation product.

In some embodiments, the fermentation product is a *Corynebacterium glutamicum* fermentation product.

In some embodiments, the *Corynebacterium glutamicum* comprises a DNA sequence set forth in SEQ ID NO:1.

Compared with other non-coding sRNA regulation methods, the non-coding sRNA obtained in the present disclosure does not require additional expression of RNA chaperone proteins. The L-leucine yield of an overexpression strain IBCLQ-257 can reach 60.2 g/L, which is improved by about 20.5% compared with that of the original strain, and the sugar-to-acid conversion rate is improved by 14.2%; after overexpressing the beneficial non-coding sRNAs, the total byproduct acids are reduced by 62.8%.

The L-isoleucine yield of an overexpression strain IBCIL-253 reaches 66.4 g/L, which is improved by 7.62% compared with that of the original strain, the sugar-to-acid conversion rate is improved by 5.23%, and the total byproduct acids are reduced by 51.7%.

The L-valine yield of strain IBCVQ-256 reaches 106.87 $\pm$ 5.80 g/L, which is improved by about 23.8% compared with that of the original strain, the sugar-to-acid conversion rate is improved by 17.9%, the production of byproduct acids is reduced by about 3.54 g/L, and the total byproduct acids are reduced by 40.8%.

The results indicate that suitable non-coding sRNAs can indeed effectively increase a fermentation concentration of the branched-chain amino acids while reducing a content of byproduct acids.

The present disclosure, by designing and introducing novel non-coding sRNA regulatory elements, not only broadens strategies of microbial metabolic engineering modification but also provides new technical means for efficiently and sustainably improving fermentation production levels of the branched-chain amino acid products.

## BRIEF DESCRIPTION OF THE DRAWINGS

The present disclosure will be further described by way of exemplary embodiments, which will be described in detail with reference to the accompanying drawings. The embodiments are not intended to be limiting. In the embodiments, the same reference numerals denote the same structures, wherein:

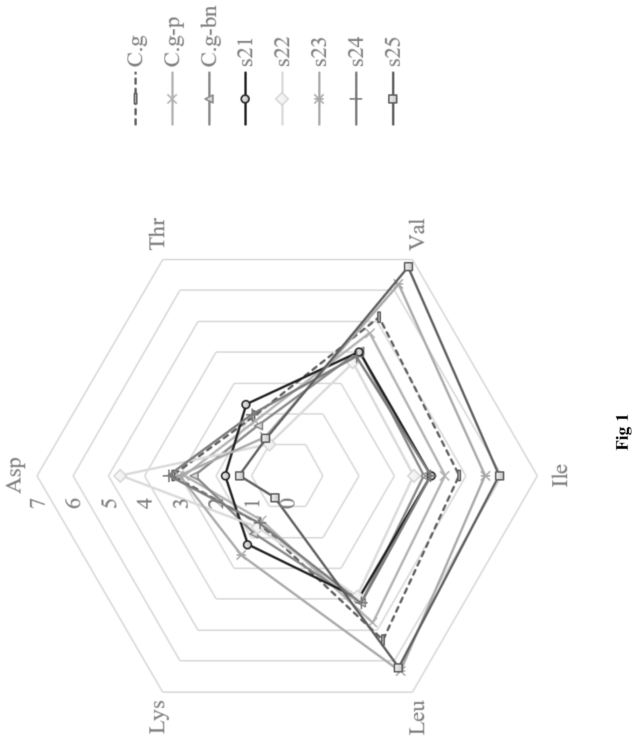

FIG. 1 is a schematic diagram illustrating accumulation of amino acid yields in *Corynebacterium glutamicum* overexpressing different non-coding sRNAs according to some embodiments of the present disclosure.

## DETAILED DESCRIPTION

In order to more clearly illustrate technical solutions of embodiments of the present disclosure, a brief introduction of the drawings required for describing the embodiments will be given below. Obviously, the drawings described below are merely some examples or embodiments of the present disclosure. For those of ordinary skill in the art, without creative efforts, the present disclosure may also be applied to other similar situations based on the drawings. Unless apparent from context or otherwise specified, the same reference numerals in the drawings denote the same structures or operations.

As shown in the present disclosure and the claims, unless the context clearly indicates otherwise, the terms "a," "an," "one," and/or "the" are not limited to singular forms and may also include plural forms. Generally, the terms "include" and "contain" merely indicate inclusion of explicitly identified operations and elements, which do not constitute an exclusive listing, and a process or a device may also include other operations or elements.

The branched-chain amino acids (BCAAs), including leucine, isoleucine, and valine, as important components in the fields of sports nutrition and health, have developed rapidly in recent years, with continuously expanding application scenarios, but their market and technologies also face some challenges. With the continuous expansion of the global market, in 2023, the global market size of the BCAAs exceeded USD 1 billion, with a compound annual growth rate (CAGR) of about 8%-10%, which is mainly driven by sports nutrition, health foods, and medical applications. The BCAAs, as supplements for anti-fatigue and promoting recovery, are widely used in fitness groups and athletes, especially in strength training and endurance sports; in addition, studies have shown potential of the BCAAs in improving insulin resistance (diabetes) and adjuvant treatment of liver diseases; and products such as BCAA beverages and functional snacks have been gradually recognized by the public and applied in daily exercise.

Currently, fermentation production strains of the BCAAs are mainly *Corynebacterium glutamicum, Brevibacter-*

*ium flavum,* and engineered *Escherichia coli.* Traditional strains have low acid production, high byproduct acid production, and low efficiency, and are gradually replaced by metabolically engineered strains. With continuous improvement of the strains, the processes thereof also need to be continuously optimized. However, at present, most research directions in fermentation of the BCAAs are still mainly concentrated in a front end, focusing on strain design with relatively little focus on processes. In addition, in related technologies, a matching degree between materials used in fermentation and processes is not well established, which further results in a phenomenon that most excellent strains have low acid production due to a lack of matching processes.

In view of the foregoing, some embodiments of the present disclosure disclose a use of the *Corynebacterium glutamicum* in L-leucine fermentation; the *Corynebacterium glutamicum* has characteristics of high L-leucine yield and low byproduct acids. In the fermentation process, in response to a cell concentration OD562 and a fermentation period, fed-batch of sugar and beet molasses is performed, and the L-leucine fermentation is carried out by controlling a ratio of the sugar and the beet molasses and a residual sugar concentration, so that a high L-leucine yield and the sugar-to-acid conversion rate are obtained and byproduct acids are reduced.

By process optimization, advantages of improved strains are amplified, thereby obtaining a process with high yield, high sugar-to-acid conversion rate, and low byproduct acids. Some embodiments of the present disclosure select relatively inexpensive molasses as materials, which provides abundant carbon and nitrogen sources, corn steep liquor with more impurities is abandoned, and the molasses is converted into a nutrient containing more carbon and nitrogen sources available for strains through hydrolysis. In addition, in order to control a carbon-to-nitrogen ratio in a fermentation broth, a more precise fed-batch process is selected, which not only ensures fermentation requirements of the strains but also effectively avoids excessively high or excessively low nitrogen content in the fermentation broth, thereby achieving goals of increasing yield and the sugar-to-acid conversion rate and reducing the byproduct acids.

One embodiment of the present disclosure provides a non-coding sRNA from *Corynebacterium glutamicum,* and an RNA sequence of the non-coding sRNA has at least 90% sequence identity to a transcription product of a DNA sequence set forth in SEQ ID NO:1

(TGCTTCATTGTTAGACAATTCTCTCTTTTTTCATTAAGGACAAAAATGAATCGAGAAAACCATTACCCGAAAAGTT

TTAAAGTTTTTTGGGCTTGGATTTTTATTCCACTCTTTG).

The *Corynebacterium glutamicum* is originally a Gram-positive bacterium isolated from soil and is widely applied to industrial production of amino acids due to its characteristic of high glutamate yield. The *Corynebacterium glutamicum,* due to its stable metabolic regulatory characteristics, has important value in fields such as food, medicine, and feed additives, for example, it is used for preparing nutritional supplements or pharmaceutical raw materials or the like. Merely by way of example, The *Corynebacterium glutamicum* may be used for amino acid production; for example, through metabolic engineering modification, the *Corynebacterium glutamicum* is optimized for efficiently producing the BCAAs (L-leucine, L-isoleucine, and L-valine).

In some embodiments, a use of the non-coding sRNA from the *Corynebacterium glutamicum* includes improving amino acid production efficiency, optimizing amino acid composition ratio, and reducing by-products.

In some embodiments, the use of the non-coding sRNA from the *Corynebacterium glutamicum* includes applications in fields such as food industry, medicine, daily chemical products, or feed additives, or the like.

In some embodiments, the use of the non-coding sRNA from the *Corynebacterium glutamicum* includes applications in preparing amino acid supplements for enhancing nutrient absorption of a human body and promoting muscle growth. The amino acid is selected from one of L-leucine, L-isoleucine, or L-valine.

In some embodiments, the RNA sequence of the non-coding sRNA from the *Corynebacterium glutamicum* has at least 95% sequence identity to the transcription product of the DNA sequence set forth in SEQ ID NO:1.

In some embodiments, the RNA sequence of the non-coding sRNA from the *Corynebacterium glutamicum* has at least 98% sequence identity to the transcription product of the DNA sequence set forth in SEQ ID NO:1.

In one aspect of embodiments of the present disclosure, the DNA sequence encoding the non-coding sRNA is set forth in SEQ ID NO:1.

One embodiment of the present disclosure provides a vector containing the DNA sequence encoding the non-coding sRNA provided in the above embodiments.

A vector is a tool used in genetic engineering for carrying exogenous DNA sequence fragments (such as target genes or regulatory sequences) into host cells. The vector includes functional elements such as an origin of replication, selectable markers (such as antibiotic resistance genes), and multiple cloning sites. The vector may be a plasmid, a virus, or other DNA molecules containing the DNA sequence encoding the non-coding sRNA.

In some embodiments, the vector containing the DNA sequence encoding the non-coding sRNA includes at least one of plasmid pXMJ19 and plasmid pXMJ. By using plasmid pXMJ19, plasmid pXMJ, or the like as the vector, and carrying the non-coding sRNA gene (SEQ ID NO:1) to regulate host metabolism, a purpose of ultimately increasing yield of the BCAAs can be achieved.

In some embodiments, construction of the vector may include the following steps:
Plasmid amplification: a backbone of pXMJ19 is amplified using high-fidelity enzymes and primers (Plasmid-F/R).

Target fragment mutagenesis: SEQ ID NO:2 (TCGTTGAGAAGTAATCAATTCTGACTTTTTTCATTAAGGA CAAAAATGAATGCAGAAAACCATTACCCGAAAAGTT TTAAAGTTTTTTGGGCTTGGATTTTTATTCCACTCTTTG) is used as a template, random mutations are introduced through error-prone PCR (primers Errp-F/R), to generate an s25 fragment (SEQ ID NO:1).

Homologous recombination and ligation: a backbone of pXMJ19 is ligated with the mutated s25 fragment after purification to form a recombinant plasmid pXMJ19-s25.

Host transformation: the recombinant plasmid is transferred into the *Corynebacterium glutamicum* (such as ATCC13002), and a recombinant strain (such as C.g-pXMJ19-s25) is obtained by screening.

One embodiment of the present disclosure provides a recombinant bacterium expressing the DNA sequence encoding the non-coding sRNA of the foregoing embodiments. A bacterial strain of the recombinant bacterium is the *Corynebacterium glutamicum.*

In some embodiments, the *Corynebacterium glutamicum* is selected from *Corynebacterium glutamicum* IBBH-15, *Corynebacterium glutamicum* IBCL-1, *Corynebacterium glutamicum* IBCVQ, *Corynebacterium glutamicum* CICC21756, *Corynebacterium glutamicum* ATCC13002, or any combination thereof.

The *Corynebacterium glutamicum* IBBH-15 is a mutant strain of the *Corynebacterium glutamicum* and is deposited at the China General Microbiological Culture Collection Center (CGMCC) in Beijing, China, with an accession number of CGMCC No. 15720, the deposit date is May 2, 2018, the depository institution is the China General Microbiological Culture Collection Center, and its address is No. 3, Yard 1, Beichen West Road, Chaoyang District, Beijing, China, Postcode: 100101.

The *Corynebacterium glutamicum* IBCL-1 (IBCL-01) is deposited in the China Center for Type Culture Collection (CCTCC) located at the College of Life Sciences, Wuhan University, Wuhan City, Hubei Province, China, with an accession number of CCTCC No. M 2022764, the deposit date is May 30, 2022, the depository institution is the China Center for Type Culture Collection, and its address is No. 299, Bayi Road, Luojiashan Street, Wuchang District, Wuhan City, Hubei Province, China, Postcode: 430072.

The *Corynebacterium glutamicum ATCC13002* is a model strain, which is obtained by purchasing its competent cell products from multiple biological reagent companies.

The *Corynebacterium glutamicum* IBCVQ may be obtained by a conventional genetic modification scheme on the basis of the model strain the *Corynebacterium glutamicum* ATCC13002, the modification including: knocking out *ldh* and *brnQ* genes on a genome of the model strain; replacing *ilvE* with an NADH-dependent leucine dehydrogenase (LeuDH) from *Lysinibacillus sphaericus,* synthesizing an *ilvN* mutant *ilvNM* (G20E, I21E, I22F, G156E) and an ilvC mutant ilvCM (S34G, L48E, and R49F), and expressing ilvBNMCM under control of the *PgapA* promoter at an *alaT* locus; expressing a *pfkA* gene under control of the *Ptac* promoter at a *ppc* locus; and expressing a *pyk* gene under control of the *Ptac* promoter at a *pta* locus.

The *Corynebacterium glutamicum* CICC21756 is an industrial strain and may be obtained by traditional mutagenesis or metabolic engineering.

In one embodiment of the present disclosure, a method for constructing a recombinant bacterium is provided. The method comprises the following steps. A recombinant strain is obtained by transferring a recombinant vector comprising a target DNA fragment into a host strain. For example, the recombinant vector including the target DNA fragment is transferred into the host strain to obtain the recombinant strain.

In some embodiments, the target DNA fragment has at least 90% sequence identity to the DNA sequence set forth in SEQ ID NO:1.

In some embodiments, the target DNA fragment has at least 95% sequence identity to the DNA sequence set forth in SEQ ID NO:1.

In some embodiments, the target DNA fragment has at least 98% sequence identity to the DNA sequence set forth in SEQ ID NO:1.

In some embodiments, the DNA sequence of the target DNA fragment is set forth in SEQ ID NO:1.

In some embodiments of the present disclosure, the recombinant bacterium expressing the DNA sequence encoding the non-coding sRNA can effectively increase the fermentation concentration of the BCAAs while reducing the content of the byproduct acids.

In one embodiment of the present disclosure, a fermentation method for a recombinant bacterium is provided. The method comprises the following steps. A seed culture is obtained by inoculating at least one recombinant strain into a seed medium for cultivation. The seed culture is inoculated into a fermentation medium for fermentation cultivation. In the fermentation cultivation, production of BCAAs is increased by adjusting at least one of an added amount of inducer, a dissolved oxygen level, pH, and nutrient supply.

The recombinant bacterium refers to a genetically modified strain obtained by introducing exogenous DNA (e.g.,

plasmids, gene fragments, or regulatory elements) into a host microorganism by genetic engineering to acquire new functions or to enhance specific metabolic capabilities. The recombinant strains may carry selectable markers (e.g., antibiotic resistance genes) and regulatory elements for expressing exogenous genes. The recombinant bacterium may include *Escherichia coli, Corynebacterium glutamicum,* and the L-leucine-producing strain.

In some embodiments, the at least one recombinant strain comprises a DNA sequence encoding the non-coding sRNA, and the DNA sequence has at least 90% sequence identity to the DNA sequence set forth in SEQ ID NO:1.

In some embodiments, the recombinant bacterium is an L-leucine-producing strain, the L-leucine-producing strain including at least one of *Corynebacterium glutamicum* mutant strains IBBH-15, IBCLQ-257, and IBCLQ-257e.

The method for obtaining the *Corynebacterium glutamicum* mutant strain IBBH-15 and the deposit information may be found in the foregoing descriptions of the present disclosure.

In the *Corynebacterium glutamicum* mutant strain IBBH-15, expressing the non-coding sRNA s25 of the foregoing embodiments yields strain IBBH-pXMJ19-s257, designated as IBCLQ-257.

At a *pta* locus of IBBH-15, expressing the non-coding sRNA s25 of the foregoing embodiments under control of a *Ptac* promoter yields strain IBBH-s257e, designated as IBCLQ-257e.

The seed culture refers to a starting culture for microbial fermentation, obtained by inoculating a small amount of strain into the seed medium for amplification so that cells reach a high-density and high-activity state, thereby providing a "seed" for subsequent large-scale fermentation. In some embodiments, the recombinant strain IBBH-15 may be inoculated into the seed medium containing 3 wt% glucose and 1.5 wt% molasses hydrolysate and cultured at 30°C until OD562 is 15 to obtain the seed culture.

The inducer refers to a chemical or physical signal for activating the expression of an exogenous gene in a host bacterium. The action mechanism of an inducer refers to releasing inhibition of gene expression by binding to a regulatory protein (e.g., a repressor in a promoter region). The inducer includes lactose, temperature, isopropyl $\beta$-D-1-thiogalacto-pyranoside (IPTG), arabinose, or the like.

The nutrients refer to compounds required for microbial growth and product synthesis, including carbon sources, nitrogen sources, inorganic salts, and growth factors, providing cells with energy, structural materials, and elements required for metabolic regulation. For example, nutrients may include glucose, molasses hydrolysate, ammonium sulfate, corn steep liquor, potassium dihydrogen phosphate, magnesium sulfate, biotin, vitamin B1, and other substances such as yeast extract, peptone, urea, sodium chloride, nucleotides, or the like.

The following examples provide specific regulation methods for one or more of an additional amount of inducer, the dissolved oxygen level, the pH, and the nutrient supply during the fermentation cultivation of the recombinant bacterium.

In some embodiments, the fermentation method for a recombinant bacterium further comprises the following steps. A dissolved oxygen level of fermentation cultivation is controlled to be 10-20% during a first 22-26 h of the fermentation cultivation, and the dissolved oxygen level is controlled to be 5-15% after the fermentation cultivation for 22-26 h. An initial pH is controlled to be 6.65-6.75. The pH is increased to 6.85-6.95 in response to a cell OD562 increasing to 19-21. The pH is increased to 6.95-7.05 in response to the cell OD562 increasing to 24-26. The pH is increased to 7.15-7.25 in response to the cell OD562 increasing to 35-37. A residual sugar concentration is controlled to be 20-30 g/L during the fermentation process.

In some embodiments, the seed medium comprises glucose at an initial concentration of 2.5-3.5 wt% and a molasses hydrolysate at an initial concentration of 1-2 wt%, the fermentation medium comprises glucose at an initial concentration of 3.5-4.5 wt% and a molasses hydrolysate at a concentration of 0.5-1.1 wt%, and the molasses hydrolysate is selected from at least one of a beet molasses hydrolysate and a cane molasses hydrolysate.

In some embodiments, the fermentation method for a recombinant bacterium further comprises the following steps. A dissolved oxygen level in the fermentation cultivation is 5-10%. In the fermentation cultivation, after a cell OD562 is increased to 19-21, the pH is increased to 6.85-6.95, and when a residual sugar concentration is decreased to 1.5-2.5 wt%, a glucose solution having a concentration of 40-50 wt% and a molasses hydrolysate having a concentration of 40-50 wt% are started to be fed. A flow rate ratio of the glucose solution to the molasses hydrolysate is controlled to be 5:1-10:1, and a ratio of the molasses hydrolysate to the fermentation medium is controlled to be 2.5-3.5 wt%. After the cell OD562 is increased to 29-31, the pH is increased to 7.05-7.15. After the cell OD562 is increased to 39-41, the pH is increased to 7.15-7.25, and the residual sugar concentration is controlled to be 1.5-2.5 wt% during the fermentation process.

Based on the same inventive concept, in one embodiment of the present disclosure, a fermentation method for a recombinant bacterium is also provided. The recombinant bacterium is an L-isoleucine-producing strain, the L-isoleucine-producing strain comprising at least one of *Corynebacterium glutamicum* mutant strains IBCIL-253, IBCL-1, and IBCIL-253k.

In the L-isoleucine-producing strain *Corynebacterium glutamicum* IBCL-1 (deposit number CCTCC NO: M 2022764, deposited on May 30, 2022), expressing the non-coding sRNA s25 provided in the embodiments of the present disclosure yields strain IBCL-pXMJ19-s253, designated as IBCIL-253.

At a *pta* locus of IBCL-1, expressing the non-coding sRNA s25 of the foregoing embodiments using a *Ptac* promoter yields strain IBCL-s253k, designated as IBCIL-253k.

The fermentation method for the recombinant bacterium provided in some embodiments of the present disclosure further comprises the following steps. When a residual sugar concentration decreases to 0.8-1.2 wt%, a supplementary sugar solution is fed to control a residual sugar concentration of the fermentation broth to be 0.8-1.2 wt%, and an inorganic salt supplementary solution is continuously fed to control a fermentation broth osmolality to be 890-910 mosm/L.

In some embodiments, the seed medium comprises glucose at an initial concentration of 4.5-5.5 wt% and a molasses hydrolysate at an initial concentration of 2.5-3.5 wt%, the fermentation medium comprises glucose at an initial concentration of 9.5-10.5 wt% and a molasses hydrolysate at a concentration of 1.5-2.5 wt%, and the molasses hydrolysate is selected from at least one of a beet molasses hydrolysate and a cane molasses hydrolysate.

In some embodiments, the fermentation method for the recombinant bacterium further comprises the following steps. A dissolved oxygen level in the fermentation cultivation is 8-12%. In the fermentation cultivation, after a cell OD562 is increased to 39-41, the pH is increased to 6.85-6.95. When a residual sugar concentration decreases to 5.5-6.5 wt%, an inorganic salt supplementary solution is fed to control a fermentation broth osmolality to be 690-710 mosm/L. When a residual sugar concentration decreases to 1.5-2.5 wt%, a supplementary solution A and a supplementary solution B are fed, the supplementary solution A comprising a glucose solution at a concentration of 40-50 wt%, a potassium dihydrogen phosphate solution at a concentration of 0.1 wt%, and a magnesium sulfate solution at a concentration of 0.05 wt%, the supplementary solution B comprising a molasses hydrolysate at a concentration of 40-50 wt%. A flow rate ratio of the supplementary solution A to the supplementary solution B is controlled to be 4:1-7:1. A ratio of the molasses hydrolysate to the fermentation medium is controlled to be 3.5-4.5 wt%. The inorganic salt supplementary solution is continuously fed to control the fermentation broth osmolality to be 890-910 mosm/L.

According to the foregoing embodiments, the present disclosure, by designing and introducing novel non-coding sRNA regulatory elements, expands strategies for microbial metabolic engineering and provides new technical means for efficiently and sustainably increasing fermentation production levels of the BCAA products.

The BCAAs, as important functional substances, have broad application value in fields such as food, pharmaceuticals, cosmetics, and feed. The BCAAs include leucine, isoleucine, and valine. Industrial production has evolved from chemical synthesis to microbial fermentation-early chemical synthesis, due to low yield and severe pollution, has been gradually replaced by microbial fermentation technology. Fermentation-based production of the BCAAs is an efficient and environmentally friendly industrial process. However, a fermentation broth contains a plurality of impurities and requires complex separation and purification processes to obtain high-purity products.

However, existing separation and purification processes face the following issues: (1) impurity separation difficulty: a fermentation broth contains impurity amino acids with similar isoelectric points (e.g., L-$\alpha$-aminobutyric acid, norvaline), and conventional ion-exchange methods have poor selectivity, resulting in insufficient purity (less than 99%). (2) Cost and environmental pressure: chromatographic equipment is expensive and has low throughput; a large dosage of the activated carbon is required for decolorizing, and waste-acid treatment costs are high. (3) Poor process continuity: existing technologies are difficult to achieve continuous production, resulting in low efficiency.

In one embodiment of the present disclosure, a separation and purification method for a fermentation product is provided, the separation and purification method comprises the following steps. The fermentation product is redissolved using p-toluenesulfonic acid, wherein a molar ratio of the fermentation product to the p-toluenesulfonic acid is 1:1-1:2. A hydrogen bond network reconstruction is performed on the redissolved fermentation product using 0.5-1.5 M inorganic acid. The fermentation product after cooling crystallization is decolorized for 30-50 min using 1-2% malic acid, 0.1-0.2% tartaric acid, and 0.5-1.5‰ activated carbon. The decolorized fermentation product is purified by ion exchange resin and evaporation crystallization to obtain a purified fermentation product.

In some embodiments, the fermentation product is the *Corynebacterium glutamicum* fermentation product. In some embodiments, the *Corynebacterium glutamicum* comprises a DNA sequence set forth in SEQ ID NO:1, and the separation and purification method comprises the following steps.

In S1, the fermentation product is redissolved using p-toluenesulfonic acid, wherein a molar ratio of the fermentation product to the p-toluenesulfonic acid is 1:1-1:2.

In some embodiments, before redissolution, a metal-polypeptide network (MPN) complex precipitation is performed, which comprises the following steps. The complex precipitation is performed on a fermentation broth containing the fermentation product using a composition of polypeptides and metal ions to precipitate the fermentation product.

The following are specific steps for performing metal-MPN complex precipitation.

(1) Preparation of an MPN reagent: a polypeptide (e.g., polyglutamic acid) and $Fe^{3+}/Zn^{2+}$ are self-assembled at a molar ratio of 1:(2-3) at a pH of 4.0-5.0 to form a soluble metal-MPN solution (a concentration of 5-10% w/v).

(2) Complexation addition: after a ceramic membrane filtration, the MPN reagent is added to a filtrate (an added amount of 0.5-2.0% v/v) and stirred for 30-60 min; target amino acids (leucine, isoleucine, valine) coordinate with the metal ions through amino groups to form an insoluble MPN-amino acid complex, while impurities are retained in a liquid phase due to charge repulsion.

(3) Separation: the precipitated complex is separated by centrifugation (3000-5000 rpm, 10-20 min) or membrane filtration, and a precipitate is collected for a subsequent redissolution step. In some embodiments of the present disclosure, by adding the soluble MPN reagent, dynamic coordination complexation is used to selectively precipitate target amino acids, replacing conventional fixed-bed adsorption and enabling equipment simplification and adaptation to high-impurity systems.

The redissolution refers to a process of redissolving a precipitated complex using an acidic reagent (e.g., p-toluenesulfonic acid, hydrochloric acid, citric acid) to release a target product and form a stable salt. Steps for the redissolution include: mixing an MPN-amino acid complex precipitate with the p-toluenesulfonic acid in proportion; releasing the target amino acids through acid dissociation; and decomposing an MPN framework under strong-acid conditions into the polypeptides and the metal ions (recoverable). The complex precipitate is mixed with the p-toluenesulfonic acid at a molar ratio of 1:(0.8-2.0), the solid content is adjusted to 40%-60%, and complete dissolution is performed at 50-60°C. The p-toluenesulfonic acid forms a stable salt with an amino group of the target amino acid via a sulfonic group, significantly increasing solubility and suppressing the formation of mixed crystals of the byproduct acids.

In S2, a hydrogen bond network reconstruction is performed on the redissolved fermentation product using 0.5-1.5 M inorganic acid.

The hydrogen bond network reconstruction refers to a process of adjusting a solution pH and ionic strength to disrupt original intermolecular hydrogen bonds and reorganize a dissolution state of the target product, thereby creating conditions for subsequent crystallization.

During the hydrogen bond network reconstruction, 0.5-2.0 M hydrochloric acid is added in stages, the system pH is adjusted to 1.0-1.5, amino acids and impurity amino acids are fully protonated, and the original hydrogen bond network is disrupted. Chloride ions (Cl-) act as ionic shielding in the solution, weakening intermolecular electrostatic attraction and facilitating subsequent separation.

In some embodiments, after the hydrogen bond network reconstruction and before decolorization, the separation and purification method further comprises the following steps. The fermentation product after the hydrogen bond network reconstruction is cooled to 9-11°C at a cooling rate of 8-12°C/h, and seed crystallization is performed for 0.5-1.5 h.

In S3, the fermentation product after cooling crystallization is decolorized for 30-50 min using 1-2% malic acid, 0.1-0.2% tartaric acid, and 0.5-1.5‰ activated carbon.

In some embodiments, gradient cooling crystallization and weak-acid complexation in combination with activated carbon adsorption are adopted.

The gradient cooling crystallization includes cooling from 55°C to 10°C at a rate of 10-15°C/h, and performing the seed crystallization at 200 rpm for 0.5-1 h, thereby suppressing formation of mixed crystals. After filtration and washing of the crystallization cake, the redissolution is performed at a solid content of 40-50%.

The weak-acid complexation in combination with the activated carbon adsorption includes adding 1%-3% malic acid and 0.5%-1.5% tartaric acid into the redissolution solution, to form complexes with the metal ions, pigment molecules, and impurity amino acids through carboxyl groups, adding the activated carbon at 0.5‰-1‰, and performing decolorization at 50-55°C for 30-40 min, followed by filtration to obtain a decolorized solution. In some embodiments of the present disclosure, by using a synergistic effect of a composite acid precipitation system (salt formation with p-toluenesulfonic acid + disruption of a hydrogen bond network with hydrochloric acid + impurity complexation with organic acids), trace-level control of impurity amino acids can be achieved, with a single species of impurity amino acid controlled to less than 0.10%. Meanwhile, a 50% reduction in the activated carbon usage can be realized, thereby reducing three-waste treatment costs and alleviating environmental protection pressure. In addition, by employing a composite acid system in combination with gradient crystallization, the formation of mixed crystals can be further avoided, the purity of a target amino acid in a crystallization cake can be improved, residual levels of impurity amino acids can be controlled, and yield can be simultaneously increased.

In S4, the decolorized fermentation product is purified by ion exchange resin and evaporation crystallization to obtain a purified fermentation product.

The ion exchange resin refers to a process of adsorbing impurities or a target product by using the charge characteristics of the ion exchange resin. The ion exchange resin includes ion-exchange separation and recycling of the p-toluenesulfonic acid: the decolorized solution is passed through ion exchange resins YN201, YN269, and YN296 at 1-2 BV/h to adsorb the p-toluenesulfonate and other organic-acid complexes. The content of the p-toluenesulfonate is monitored, and feeding is stopped when the content in the effluent exceeds 100 ppm. The water rinsing is performed until the solid content is 0, and gradient elution is performed with 1-3 M hydrochloric acid to regenerate the resin column, thereby achieving recovery and reuse of the p-toluenesulfonic acid. In some embodiments of the present disclosure, recycling of the p-toluenesulfonic acid is achieved by gradient elution with hydrochloric acid at different concentrations, thereby further reducing wastewater treatment costs and environmental protection pressure.

The evaporation crystallization: the effluent is subjected to the evaporation crystallization, and a crystallization cake of a high-purity amino acid is obtained after filtration.

In some embodiments, after ion exchange resin and before evaporation crystallization, the separation and purification method further comprises the following steps. The p-toluenesulfonic acid is eluted and recovered using 0.3-0.8 M hydrochloric acid, and the resin is regenerated using 2.5-3.5 M hydrochloric acid, thereby reducing costs and environmental burden. In some embodiments of the present disclosure, the separation and purification method for the fermentation product, without changing equipment, resin, and processing technology, can be applicable to the separation and purification of three BCAAs, requiring a small equipment investment and exhibiting strong process applicability and a high degree of continuity, making it suitable for industrial scale-up production. It should be noted that the separation and purification method for the fermentation product described in embodiments of the present disclosure is not only applicable to the separation and purification of the fermentation product of *Corynebacterium glutamicum* described above, but also to the separation and purification of fermentation products of other strains, which is not limited herein.

The fermentation method described above is described in detail below through a plurality of examples and comparative examples. It should be noted that reaction conditions, reaction materials, and amounts of the reaction materials in the examples are provided for illustration and do not limit the scope of the present disclosure. The comparative examples are the control groups of the examples.

In the present disclosure, unless otherwise expressly stated, percentages and percentage contents are by mass. Unless specially stated, experimental methods used are conventional methods, and materials and reagents used are commercially available.

**Example 1: Construction of an sRNA regulatory system for engineered *Corynebacterium glutamicum***

The plasmid pXMJ19 was amplified using plasmid primers Plsmid-F/R and a high-fidelity DNA polymerase; a DNA fragment s25 (SEQ ID NO:1) was amplified using error-prone PCR primers Errp-F/R and the high-fidelity DNA polymerase. After purification, the two fragments were assembled by homologous recombination, and the resulting vector was transformed into the *Corynebacterium glutamicum* model strain ATCC13002 to obtain strain C.g-pXMJ19-s25, designated as s25.

In some embodiments, SEQ ID NO:1 (s25) is obtained from SEQ ID NO:2 by error-prone PCR mutagenesis and sequence-screening-based optimization. Merely by way of example, the DNA fragment s25 (SEQ ID NO:1) may be obtained as follows:

(1) The plasmid pXMJ19 was amplified using the plasmid primers Plsmid-F/R and the high-fidelity DNA polymerase. A DNA fragment bn (SEQ ID NO:2) was amplified using error-prone PCR primers Errp-F/R and the high-fidelity DNA polymerase. After purification, the two fragments were assembled by homologous recombination to obtain a vector pXMJ19-bn.

(2) Using the plasmid pXMJ-bn as the template, an sRNA-specific portion was amplified with an error-prone PCR polymerase. A reaction system is as shown in Table 2, and a reaction program is as shown in Table 2. To improve the mutation rate, after a first round of error-prone PCR was completed, the plasmid template was digested; and a purified product was used as the template to perform error-prone PCR again, cycling three times. After PCR was completed, agarose gel electrophoresis was performed; under a blue-light real-time imager, it was determined whether a size of a target gene fragment was correct; if a band size was correct, 5 $\mu$L of a DMT enzyme was added into the system and incubated at 37°C for 15 min; after digestion was finished, the product was purified and recovered.

(3) Using the plasmid pXMJ-sRNAbn as the template, the remaining fragment was amplified with the high-fidelity DNA polymerase. The reaction system is as shown in Table 3, and a reaction program is as shown in Table 2. After purification, the two fragments were assembled by homologous recombination, and the resulting mutant plasmid was transformed into the starting strain (i.e., the host strain) described below. Strains exhibiting improved fermentation performance were subjected to sequencing. Among six groups, only one group shows a more pronounced effect, and a mutant sequence of the group is set forth in SEQ ID NO:1, designated as s25. Additionally, the plasmid pXMJ19-bn was transformed into the *Corynebacterium glutamicum ATCC13002* to obtain strain C.g-pXMJ-bn, designated as C.g-bn, which served as a control for subsequent experiments. Using the same transformation method, the plasmid pXMJ19 was transformed into the *Corynebacterium glutamicum* to obtain strain C.g-p, which also served as a control for subsequent experiments.

Table 1 Error-prone PCR Reaction System

| Reaction Component | Volume (50 μL) |
|---|---|
| Template | 1 μL |
| Error-Prone PCR Primer F | 1.5 μL |
| Error-Prone PCR Primer R | 1.5 μL |
| 2× *Taq* DNA Polymerase mixture | 25 μL |
| MnCl$_2$ (5 mM) | 2 μL |
| DMSO | 2 μL |
| ddH$_2$O | 17 μL |

Table 2 Error-prone PCR Reaction Program

| Step | Cycle Number | Temperature | Amplification Time |
|---|---|---|---|
| 1 | 1 | 94°C | 3 min |
| 2 | 25-30 | 94°C | 30 sec |
| | | 57°C | 30 sec |
| | | 72°C | 1 min/kb |
| 3 | 1 | 72°C | 10 min |
| 4 | 1 | 4°C | Hold |

Table 3 PCR Reaction System for Amplifying Other Fragments of pXMJ19-sRNAbn

| Reagent | Volume |
|---|---|
| 2×ApexHFFS PCR Master Mix | 25 μL |
| Template DNA | 1 μL |
| Plasmid Primer F | 1 μL |
| Plasmid Primer R | 1 μL |
| ddH$_2$O | 22 μL |

Plasmid primer Plsmid-F: ggctgttttggcggatgagag; SEQ ID NO:3.

Plasmid primer Plsmid-R: ttgttatccgctcacaattccac; SEQ ID NO:4.

Error-prone PCR primer Errp-F: gtggaattgtgagcggataacaa; SEQ ID NO:5.

Error-prone PCR primer Errp-R: ctctcatccgccaaaacagcc; SEQ ID NO:6.

**Example 2: Determination of acid producing performance of sRNA overexpression strains from *Corynebacterium glutamicum***

The *Corynebacterium glutamicum* model strain ATCC13002 (C.g), the empty vector overexpression strain C.g-p, and the *Corynebacterium glutamicum* overexpressing different non-coding RNAs (C.g-bn, s21-s25) were inoculated into a shake-flask medium and cultured at 37°C and 100 rpm for 16 h until the OD562 of about 15 was reached, to obtain cultures for subsequent use. The cultures were then inoculated into the shake-flask medium at 9% (V/V) and subjected to oscillatory cultivation at 37°C and 120 rpm for 68 h. At 48 h, 1 mM lactose was added to induce plasmid expression. The fermentation was completed after 68 h.

FIG. 1 is a schematic diagram illustrating accumulation of amino acid yields in *Corynebacterium glutamicum* overexpressing different non-coding sRNAs according to some embodiments of the present disclosure. In the FIG. 1, Asp refers to aspartic acid, Lys refers to lysine, Thr refers to threonine, Leu refers to leucine, Ile refers to isoleucine, and Val refers to valine. As shown in FIG. 1, it can be seen from the yield results that strains overexpressing the empty vector and the template sRNA exhibit an overall reduction in yield. The reduction in yield of the empty vector overexpressing strain is likely to be attributed to a decrease in overall capacity of the acid production caused by the burden of the vector replication, while the reduction in yield of the strain overexpressing the template sRNA is attributed to a decline in a capacity of the acid production caused due to an adverse effect exerted by the template sRNA on the acid production. For the other five obtained non-coding sRNAs, different effects on the amino acid production capacity are observed. Compared with the strain overexpressing the empty vector, the strain overexpression the non-coding sRNA s21 enhances accumulation of threonine and lysine while reducing accumulation of aspartic acid and the BCAAs, including L-valine, L-leucine, and L-isoleucine, in the fermentation broth. In the strain overexpressing the non-coding sRNA s22, accumulation of aspartic acid is markedly increased, whereas yields of threonine, L-valine, L-leucine, and L-isoleucine are significantly reduced. In the strain overexpressing the non-coding sRNA s23, accumulation of aspartic acid and threonine shows slight fluctuations, yields of L-valine, L-leucine, and L-isoleucine are significantly increased, and accumulation of lysine is also enhanced. The strain overexpressing non-coding sRNA s24 shows no obvious benefit in improving yields of the detected amino acids. In the strain overexpressing the non-coding sRNA s25, accumulation of aspartic acid and threonine is reduced, whereas yields of L-valine, L-leucine, and L-isoleucine are significantly increased, and accumulation of lysine is reduced. In particular, this beneficial effect also surpasses the adverse effect caused by vector burden, with yields of L-valine, L-isoleucine, and L-leucine increased to 1.32-fold, 1.17-fold, and 1.24-fold of that of the model strain, respectively.

**Example 3: Effect of sRNA s25 expression in L-leucine-producing strains on acid production**

In the *Corynebacterium glutamicum* mutant strain IBBH-15 (deposited at the China General Microbiological Culture Collection Center (CGMCC) in Beijing, China, with the accession number of CGMCC No. 15720 and a deposit date of May 2, 2018), the non-coding sRNA s25 described in the examples above was expressed to obtain strain IBBH-pXMJ19-s25-7, designated as IBCLQ-257. In the L-leucine-producing strain, expression of the empty vector pXMJ19 was performed, designated as IBBH-15-p; and expression of pXMJ-sRNAbn was performed, designated as IBBH-15-bn.

One loop of slant bacterial lawn from each of the L-leucine-producing strain IBBH-15, the strain carrying the empty plasmid IBBH-15-p, the overexpression strain IBBH-15-bn, and the overexpression strain IBCLQ-257 was respectively inoculated into four 3 L shake flasks. The shake flasks were cultivated at 80 rpm and 30°C until the OD562 reached approximately 15, to obtain shake-flask seed cultures for later use.

The shake-flask seed culture was inoculated into a 30 L fermentation tank fermentation medium at an inoculation amount of 10% (V/V). At 48 h, 1 mM lactose was added to induce plasmid expression. The initial temperature was 30°C, and the initial aeration rate was 0.8 L/min. Dissolved oxygen was controlled to be 10-20% during the first 24 h of the fermentation process and 5-15% after 24 h by adjusting the stirring speed and the aeration rate. The pH of the fermentation process was controlled by automatically feeding ammonia water. The initial pH was controlled at 6.7; after a cell OD562 increased to 20, the pH was increased to 6.9; after a cell OD562 increased to 25, the pH was increased to 7.0; and after a cell OD562 increased to 36, the pH was increased to 7.2. During the fermentation process, the residual sugar concentration was controlled to be 20-30 g/L.

Table 4 Main Results of Example 3

| Strain | Yield g/L | Sugar-to-Acid Conversion Rate % | Byproduct Acid g/L |
|---|---|---|---|
| IBBH15 | 49.8±2.13 | 24.6% | 2.58±0.35 |
| IBBH15-p | 49.7±1.88 | 24.2% | 2.68±0.33 |
| IBBH15-bn | 43.2±2.23 | 21.0% | 1.36±0.41 |
| IBCLQ-257 | 60.02±2.01 | 27.7% | 0.96±0.15 |

The data from Table 4 shows that the strain IBCLQ-257 overexpressing sRNA s25 exhibits an approximately 20.5% increase in L-leucine yield compared with the control strain IBBH-15, while byproduct acids are significantly reduced. In contrast, the strain IBBH-15-bn shows visible decreases in both acid production and byproduct acid formation compared with the original starting strain and the empty-vector control strain IBBH-15-p. The results from Table 4 indicate that the influence of the non-coding sRNA on regulation of metabolic acid production is related to the sRNA sequence.

Initial fermentation medium of the fermentation tank:
Glucose 100 g/L, ammonium sulfate 5 g/L, corn steep liquor 10 mL/L, potassium dihydrogen phosphate 5 g/L, magnesium sulfate 2.5 g/L, ferrous sulfate 0.01 g/L, manganese sulfate 0.01 g/L, biotin 100 $\mu$g/L, vitamin B1 300 $\mu$g/L, L-methionine 0.1 g/L, L-isoleucine 0.1 g/L, L-glutamic acid 0.1 g/L, vegetable oil 1 mL/L, pH 7.0-7.2.

Feeding sugar solution: glucose 400 g/L, magnesium sulfate 0.5 g/L.

**Example 4: Effect of sRNA s25 expression in L-isoleucine-producing strains on acid production**

In the L-isoleucine-producing strain *Corynebacterium glutamicum* IBCL-1 (with an accession number CCTCC No: M 2022764, deposited on May 30, 2022), the non-coding sRNA s25 described in Example 2 was expressed to obtain strain IBCL-pXMJ19-s253, designated as IBCIL-253. In the L-isoleucine-producing strain, expression of the empty vector pXMJ19 was performed, designated as IBCL-1-p; and expression of pXMJ-sRNAbn was performed, designated as IBCL-1-bn.

The one loop of slant bacterial lawn from each of the L-isoleucine-producing strain IBCL-1, the strain carrying the empty plasmid IBCL-1-p, the overexpression strain IBCL-1-bn, and the overexpression strain IBCIL-253 was respectively inoculated into two 3-L shake flasks. The shake flasks were cultivated at 30°C and 90 rpm for 16 h until the OD562 reached approximately 15, to obtain the shake-flask seed cultures for later use. The shake-flask seed culture was inoculated into the 30-L fermentation tank fermentation medium at an inoculation amount of 10% (V/V). At 18 h, 1 mM lactose was added to induce plasmid expression. The initial temperature was 30°C, and the pH was 6.8. During the fermentation process, aeration and stirring speed were continuously adjusted to control the dissolved oxygen at around 10%. At 48 h, 1 mM lactose was added again to induce plasmid expression. When the cell OD562 of the fermentation broth increased to approximately 40, the pH was adjusted to approximately 7.0. When the residual sugar concentration of the fermentation broth decreased to 6%, feeding of the inorganic salt supplementary solution was started to control the fermentation broth osmolality at approximately 700 mosm/L. When the residual sugar concentration decreased to below 1%, feeding of the supplementary sugar solution was started to control the residual sugar concentration of the fermentation broth at approximately 1%, while continuing feeding of the inorganic salt supplementary solution to control the fermentation broth osmolality at approximately 900 mosm/L. The fermentation was completed upon exhaustion of sugar after approximately 30 h. The fermentation yield is shown in Table 5.

Table 5 Main Results of Example 4

| Strain | Yield g/L | Sugar-to-Acid Conversion Rate % | Byproduct Acid g/L |
|---|---|---|---|
| IBCL-1 | 61.7±1.40 | 36.3% | 1.74±0.40 |
| IBCL-1-p | 60.7±2.88 | 35.7% | 1.93±0.50 |
| IBCL-1-bn | 56.6±5.86 | 33.3% | 1.64±0.37 |
| IBCIL-253 | 66.4±3.20 | 38.2% | 0.84±0.12 |

It can be seen from Table 5 that the strain IBCIL-253 overexpressing sRNA s25 exhibits an approximately 7.6% increase in L-isoleucine yield compared with the control strain IBCL-1, while byproduct acids are reduced by 51.7%. In contrast, the strain IBCL-1-bn shows marked decreases in both acid production and byproduct acid formation compared with the original starting strain IBCL-1. The results indicate that the influence of the non-coding sRNA on the regulation of metabolic acid production is related to the sRNA sequence.

Seed medium:

Glucose 30 g/L, corn steep liquor powder 10 g/L, ammonium sulfate 5 g/L, potassium dihydrogen phosphate 1 g/L, magnesium sulfate 0.5 g/L, calcium carbonate 10 g/L, pH 6.7-7.2; dispensed at 500 mL/5000 mL; sterilization condition 121°C/20 min.

Fermentation medium:

Glucose 100 g/L, corn steep liquor powder 8 g/L, ammonium sulfate 5 g/L, potassium dihydrogen phosphate 1 g/L, magnesium sulfate 0.5 g/L;

Supplementary sugar solution: glucose 400 g/L, potassium dihydrogen phosphate 1 g/L, magnesium sulfate 0.5 g/L.

Inorganic salt supplementary solution: ammonium sulfate 100 g/L.

**Example 5: Effect of sRNA s25 expression in L-valine-producing strains on acid production**

Based on the model strain *Corynebacterium glutamicum ATCC13002* (competent cell products of which are commercially available from various biological reagent companies), genetic modifications were carried out according to a conventional genetic modification scheme to obtain the *Corynebacterium glutamicum* IBCVQ. The modifications included: knocking out the *ldh* and *brnQ* genes on the genome of the model strain; replacing *ilvE* with an NADH-dependent leucine dehydrogenase (LeuDH) from *Lysinibacillus sphaericus,* synthesizing an *ilvN* mutant *ilvNM* (G20E, I21E, I22F, G156E) and an *ilvC* mutant *ilvCM* (S34G, L48E, and R49F), and expressing *ilvBNMCM* under the *PgapA* promoter at the *alaT* locus; expressing the *pfkA* gene under the *Ptac* promoter at the *ppc* locus; and expressing the *pyk* gene under the *Ptac* promoter at the *pta* locus.

In the L-valine-producing strain IBCVQ, the non-coding sRNA s25 described in Example 4 was expressed to obtain strain IBCVQ-pXMJ19-s256, designated as IBCLQ-256. In the L-valine-producing strain, expression of the empty vector pXMJ19 was performed, designated as IBCVQ-p; and expression of pXMJ-sRNAbn was performed, designated as IBCVQ-bn.

The one loop of slant bacterial lawn from each of L-valine-producing strain IBCVQ, the strain carrying the empty plasmid IBCVQ-p, the overexpression strain IBCVQ-bn, and the overexpression strain IBCVQ-256 was respectively inoculated into two 3-L shake flasks. The shake flasks were cultivated at 120 rpm and 30°C for 16-18 h until the OD562 reached approximately 15, to obtain the shake-flask seed cultures for later use. The shake-flask seed culture was inoculated into the 30 L fermentation tank fermentation medium at the inoculation amount of 10% (V/V). From 0 h to 20 h, the cultivation conditions were: temperature 30°C, tank pressure 0.02-0.03 MPa, after the dissolved oxygen naturally decreased to around 20%, the stirring speed and aeration rate being adjusted to control the dissolved oxygen to be not lower than 20%. From 20 h to 48 h, the cultivation conditions were: temperature 31°C, tank pressure 0.04-0.08 MPa, and the dissolved oxygen being controlled at 1% $\pm$ 0.1% by adjusting the stirring speed and aeration rate. During the fermentation process, the residual sugar concentration of the fermentation broth was controlled to be 20-30 g/L.

After 48 h of fermentation, the L-valine yield, conversion rate, and a content of the byproduct acid are shown in Table 6.

Table 6 Main Results of Example 5

| Strain | Yield g/L | Sugar-to-Acid Conversion Rate % | Byproduct Acid g/L |
|---|---|---|---|
| IBCVQ | 86.3$\pm$3.40 | 44.8% | 8.67$\pm$2.14 |
| IBCVQ-p | 82.5$\pm$2.31 | 42.8% | 8.12$\pm$1.79 |
| IBCVQ-bn | 81.2$\pm$3.25 | 42.2% | 7.2$\pm$1.49 |
| IBCVQ-256 | 106.84$\pm$5.80 | 52.8% | 5.13$\pm$0.72 |

It can be seen from Table 6 that the strain IBCVQ-256 overexpressing sRNA s25 exhibits a 23.8% increase in L-valine yield compared with the control strain IBCVQ, while the total byproduct acid decreases by 40.8%. In contrast, the strain IBCVQ-bn shows decreases in both acid production and byproduct acid formation compared with the original starting strain IBCVQ. The results indicate that the influence of the non-coding sRNA on the regulation of metabolic acid production is related to the sRNA sequence.

Slant medium: Urea 2.5 g/L, ammonium sulfate $(NH_4)_2SO_4$ 5 g/L, $KH_2PO_4$ 0.5 g/L, $K_2HPO_4$ 0.5 g/L, $MgSO_4·7H_2O$ 0.5 g/L, yeast extract powder 1.8 g/L, peptone 5 g/L, $FeSO_4·7H_2O$ 6 mg/L, $MnSO_4·H_2O$ 4 mg/L, biotin 0.2 mg/L, vitamin B1 0.2 mg/L, glucose 40 g/L, agar powder 15 g/L.

Fermentation medium: ammonium sulfate $(NH_4)_2SO_4$ 8 g/L, $KH_2PO_4$ 1 g/L, $K_2HPO_4$ 1 g/L, $MgSO_4·7H_2O$ 0.5 g/L, yeast powder 3.5 g/L, $FeSO_4·7H_2O$ 10 mg/L, $MnSO_4·H_2O$ 4 mg/L, biotin 0.02 mg/L, vitamin B1 2 mg/L, vitamin B3 30 mg/L, vitamin B6 8 mg/L, glucose 90 g/L, vegetable oil 40 mL/L.

Feeding medium: glucose 400 g/L.

**Example 6: Preferred example of Leucine fermentation**

1. Composition of the seed medium:

Primary seed medium: glucose 3 wt%, enzymatic hydrolysate of beet molasses 1.5 wt%, ammonium sulfate 0.5 wt%, potassium dihydrogen phosphate 0.1 wt%, magnesium sulfate 0.05 wt%, calcium carbonate 1 wt%, pH 6.7-7.2; dispensed at 500 mL/5000 mL; sterilization condition 121°C/20 min.

Secondary seed tank medium: glucose 3 wt%, enzymatic hydrolysate of beet molasses 1.5 wt%, ammonium sulfate 0.5 wt%, potassium dihydrogen phosphate 0.1 wt%, magnesium sulfate 0.05 wt%, pH 6.8-7.2; sterilization condition 121°C/20 min.

2. The fermentation medium:

Initial fermentation medium in the Standard fermentation tank: glucose 4 wt%, beet molasses hydrolysate 0.8 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%; supplementary solution B: beet molasses hydrolysate 40-50 wt%. During use, concentrations of supplementary solution A and supplementary solution B are required to be kept consistent.

3. The fermentation tank process (500 L):

One loop of each of the *Corynebacterium glutamicum* IBCLQ-257, IBCLQ-257e, and IBBH-15 was respectively inoculated into the primary seed medium in shake flasks and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated into 50 L of the secondary seed tank medium at 0.2% (V/V). Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained.

The secondary seed culture was inoculated into the 500 L fermentation tank at 10%, with a fermentation volume of 300 L. Fermentation was carried out at 30°C with pH 6.7, the stirring speed of 200 rpm, an aeration rate of 60 L/min, and a tank pressure of 0.05 MPa. The stirring speed and aeration were alternately adjusted to maintain dissolved oxygen at 5-10%. After the cell OD562 increased to 20, the pH was increased to 6.9. When the residual sugar concentration decreased to 2 wt%, feeding was started, and at this time, the flow rate ratio of the supplementary solution A and the supplementary solution B was controlled to be 8:1. After the cell OD562 increased to 30, the pH was increased to 7.1, and 1 mM lactose was added at one time (no addition was required for the strain IBBH-15). After the cell OD562 increased to 40, the pH was increased to 7.2. The residual sugar concentration was controlled at 2 wt%, and the fermentation broth was discharged after the supplementary sugar was exhausted.

Table 7 Main Results of Example 6

| Strain | L-Leucine Yield wt% | Sugar-to-Acid Conversion Rate % | Byproduct Acid wt% |
|---|---|---|---|
| IBCLQ-257 | 6.65 | 33.25 | 0.058 |
| IBCLQ-257e | 6.34 | 31.70 | 0.076 |
| IBBH15 | 5.57 | 27.85 | 0.15 |

As shown in Example 6, the core of the present disclosure is that the beet molasses is enzymatically hydrolyzed using invertase and papain to obtain the beet molasses hydrolysate. During L-leucine fermentation, the glucose solution and the beet molasses hydrolysate are co-fed at a certain ratio to control the residual sugar concentration. In this way, the residual sugar concentration can be controlled, and the total nitrogen supply can be controlled through feeding of an organic nitrogen source, thereby preventing excessively rapid cell growth. After enzymatic hydrolysis of the beet molasses, sucrose is completely hydrolyzed into the glucose and the fructose, which are more readily utilized by cells, and papain hydrolyzes macromolecular proteins to enable full utilization of the nitrogen source, thereby achieving complete utilization of both the carbon source and the nitrogen source of molasses.

In the fermentation process, the acid production refers to the actual concentration of L-leucine in the fermentation broth. The calculation formula of the sugar-to-acid conversion rate is as follows:

Sugar-to-acid conversion rate = (actual concentration of L-leucine × actual fermentation volume) / (total actual consumption of glucose × 100%)

Simultaneously, the byproduct acids refer to the total of valine, lysine, alanine, and glutamic acid in the fermentation broth. These two parameters are jointly used to evaluate the efficiency of the fermentation process and the composition of the products.

In summary, the *Corynebacterium glutamicum* IBCLQ-257, IBCLQ-257e, and IBBH-15 exhibit significant advantages in L-leucine yield, sugar-to-acid conversion rate, and the byproduct acids in the fermentation process described in Example 6.

**Example 7: Determination of initial added amount range**

1. The seed formulation and cultivation process are the same as those in Example 6.

2. The fermentation medium:

Initial fermentation medium in the standard fermentation tank: glucose 4 wt%, beet molasses hydrolysate 0.5-1.1 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A glucose 40-50 wt%; supplementary solution B beet molasses hydrolysate 40-50 wt%. During use, concentrations of the supplementary solution A and the supplementary solution B are required to be kept consistent.

3. The fermentation tank process (500 L):

One loop of each of the *Corynebacterium glutamicum* IBCLQ-257, IBCLQ-257e, and IBBH-15 was respectively inoculated into the primary seed medium in shake flasks and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated into 50 L of the secondary seed tank medium at 0.2% (V/V). Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained. The secondary seed culture was inoculated into the 500-L fermentation tank at 10%, with an initial fermentation filling volume of 60%. Fermentation was carried out at 30°C with pH 6.7, a stirring speed of 200 rpm, an aeration rate of 60 L/min, and a tank pressure of 0.05 MPa. The stirring speed and aeration rate were alternately adjusted to maintain dissolved oxygen at 5-10%. After the cell OD562 increased to 20, the pH was increased to 6.9. When the residual sugar concentration decreased to 2 wt%, feeding was started, and at this time, the flow rate ratio of the supplementary solution A and the supplementary solution B was controlled to be 8: 1. After the cell OD562 increased to 30, the pH was increased to 7.1, and 1 mM lactose was added at one time (no addition was required for the strain IBBH-15). After the cell OD562 increased to 40, the pH was increased to 7.2. The residual sugar concentration was controlled at 2 wt%, and the fermentation broth was discharged after the feeding sugar was exhausted.

Table 8 Main Results of Example 7

| Strain | Initial Concentration (wt%) | L-Leucine Yield (wt%) | Sugar-to-Acid Conversion Rate (%) | Byproduct Acid (wt%) |
|---|---|---|---|---|
| IBCL Q-257 | 0.5 | 6.52 | 32.6 | 0.06 |
| | 0.8 | 6.55 | 32.75 | 0.09 |
| | 1.1 | 6.53 | 32.65 | 0.1 |
| IBCL Q-257e | 0.5 | 6.24 | 31.2 | 0.09 |
| | 0.8 | 6.25 | 31.25 | 0.12 |
| | 1.1 | 6.17 | 30.85 | 0.11 |
| IBBH 15 | 0.6 | 5.48 | 27.4 | 0.17 |
| | 0.7 | 5.32 | 26.6 | 0.18 |
| | 0.9 | 5.33 | 26.65 | 0.16 |

The ratio A/B of the supplementary solution A to the supplementary solution B refers to the volume ratio of supplementary solution A to supplementary solution B during feeding. Since the concentrations of the supplementary glucose solution and the supplementary beet molasses hydrolysate are basically consistent, the ratio may also be regarded as the mass ratio of the supplementary glucose solution to the supplementary beet molasses hydrolysate during feeding.

As shown in Example 7, the present example performs optimization of the initial concentration of the beet molasses hydrolysate. Verification under different initial concentration conditions shows that the initial added amount is within a range of 0.5-1.1 wt%.

**Example 8: Determination of ratio of glucose to molasses during supplementary feeding**

1. The seed formulation and cultivation process are the same as those in Example 6.

2. The fermentation medium:

Initial fermentation medium in the standard fermentation tank: glucose 4 wt%, molasses hydrolysate 0.8 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%; supplementary solution B: beet molasses hydrolysate 40-50 wt%. During use, concentrations of supplementary solution A and supplementary solution B are required to be kept consistent.

3. Fermentation tank process (500 L):

One loop of each of the *Corynebacterium glutamicum* IBCLQ-257, IBCLQ-257e, and IBBH-15 was respectively inoculated into the primary seed medium in shake flasks and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated into 50 L of the secondary seed tank medium at 0.2% (V/V). Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained. The secondary seed culture was inoculated into the 500-L fermentation tank at 10%, with an initial fermentation filling volume of 60%. Fermentation was carried out at 30°C with pH 6.7, a stirring speed of 200 rpm, an aeration rate of 60 L/min, and a tank pressure of 0.05 MPa. The stirring speed and aeration were alternately adjusted to maintain dissolved oxygen at 5-10%. After the cell OD562 increased to 20, the pH was increased to 6.9. When the residual sugar concentration decreased to 2 wt%, feeding was started, and at this time, the flow rate ratio of the supplementary solution A and the supplementary solution B was controlled to be 6:1 to 10:1. After the cell OD562 increased to 30, the pH was increased to 7.1, and 1 mM lactose was added at one time (no addition is required for the strain IBBH-15). After the cell OD562 increased to 40, the pH was increased to 7.2. If the molasses hydrolysate was exhausted first, feeding of the supplementary glucose solution was continued. If the supplementary glucose solution was exhausted first, feeding of the beet molasses hydrolysate was not continued. The residual sugar concentration was controlled to be 2 wt%, and the fermentation broth was discharged after the supplementary glucose solution was exhausted.

Table 9 Main Results of Example 8

| Strain | A/B | L-Leucine wt% | Sugar-to-Acid Conversion Rate % | Byproduct Acid wt% |
|---|---|---|---|---|
| IBCLQ-257 | 6:1 | 6.33 | 31.65 | 0.09 |
| | 7:1 | 6.45 | 31.1 | 0.08 |
| | 10:1 | 6.35 | 30.55 | 0.08 |
| IBCLQ-257e | 6:1 | 6.02 | 30.1 | 0.13 |
| | 9:1 | 6.08 | 30.4 | 0.1 |
| | 10:1 | 6.05 | 30.25 | 0.11 |
| IBBH15 | 6:1 | 5.25 | 25.85 | 0.16 |
| | 7:1 | 5.35 | 26.25 | 0.15 |
| | 10:1 | 5.23 | 25.7 | 0.18 |

As shown in Example 8, the present example performs optimization of the ratio of supplementary solutions A and B. The results show that in a range of 6:1 to 10:1, an ideal effect can be achieved, which also indicates that the ratio between glucose and molasses during supplementary feeding needs to be strictly controlled to be within the range.

**Example 9: Determination of cane molasses hydrolysate being used in the current process**

1. The seed formulation and cultivation process are the same as those in Example 6.

2. The fermentation medium:

Initial fermentation medium in the standard fermentation tank: glucose 4 wt%, (beet/cane) molasses hydrolysate 0.8 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%; supplementary solution B: beet molasses hydrolysate 40-50 wt%. During use, concentrations of supplementary solution A and supplementary solution B are required to be kept consistent.

3. The fermentation tank process (500 L):

One loop of each of the *Corynebacterium glutamicum* IBCLQ-257, IBCLQ-257e, and IBBH-15 was respectively inoculated into the primary seed medium in shake flasks and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated into 50 L of the secondary seed tank medium at 0.2% (V/V). Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained. The secondary seed culture was inoculated into the 500-L fermentation tank at 10%, with an initial fermentation filling volume of 60%. Fermentation was carried out at 30°C with pH 6.7, a stirring speed of 200 rpm, an aeration rate of 60 L/min, and a tank pressure of 0.05 MPa. The stirring speed and aeration rate were alternately adjusted to maintain dissolved oxygen at 5-10%. After the cell OD562 increased to 20, the pH was increased to 6.9. When the residual sugar concentration decreased to 2 wt%, feeding was started, and at this time, the flow rate ratio of the supplementary solution A and the supplementary solution B was controlled to be 8: 1. After the cell OD562 increased to 30, the pH was increased to 7.1, and 1 mM lactose was added at one time (no addition was required for the strain IBBH-15). After the cell OD562 increased to 40, the pH was increased to 7.2. The residual sugar concentration was controlled at 2 wt%, and the fermentation broth was discharged after the feeding sugar was exhausted.

Table 10 Main Results of Example 9

| Strain | Cane Molasses Hydrolysate Percentage % | Beet Molasses Hydrolysate Percentage % | L-Leucine wt% | Sugar-to-Acid Conversion Rate % | Byproduct Acid wt% |
|---|---|---|---|---|---|
| IBCLQ-257 | 100 | 0 | 6.35 | 31.75 | 0.07 |
| | 50 | 50 | 6.41 | 32.05 | 0.07 |
| | 20 | 80 | 6.52 | 32.6 | 0.08 |
| IBCLQ-257e | 90 | 10 | 6.11 | 30.55 | 0.11 |
| | 50 | 50 | 6.15 | 30.75 | 0.09 |
| | 20 | 80 | 6.24 | 31.2 | 0.1 |
| IBBH15 | 100 | 0 | 5.46 | 27.3 | 0.15 |
| | 80 | 20 | 5.35 | 26.75 | 0.16 |
| | 10 | 90 | 5.52 | 27.6 | 0.15 |

As shown in Example 9, cane molasses hydrolysate is used in combination with the beet molasses hydrolysate in the present example. The cane molasses hydrolysate may replace the beet molasses hydrolysate, but the effect shows a slight difference compared with the beet molasses hydrolysate.

Example 9 proves that the hydrolysis method and the supplementary feeding manner are applicable to the cane molasses for leucine fermentation.

**Example 10: Determination of partially hydrolyzed molasses being used for fermentation**

1. The seed formulation and cultivation process are the same as those in Example 6.

2. The fermentation medium:

Initial fermentation medium in the standard fermentation tank: glucose 4 wt%, partially hydrolyzed molasses hydrolysate 0.7 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%; supplementary solution B: beet molasses hydrolysate 40-50 wt%. During use, concentrations of the supplementary solution A and the supplementary solution B are required to be kept consistent.

3. The fermentation tank process (500 L):

One loop of each of the *Corynebacterium glutamicum* IBCLQ-257, IBCLQ-257e, and IBBH-15 was respectively inoculated with one loop into the primary seed medium in shake flasks and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated into 50 L of the secondary seed tank medium at 0.2% (V/V). Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained. The secondary seed culture was inoculated into the 500-L fermentation tank at 10%, with an initial fermentation filling volume of 60%. Fermentation was carried out at 30°C with pH 6.7, a stirring speed of 200 rpm, an aeration rate of 60 L/min, and a tank pressure of 0.05 MPa. The stirring speed and aeration rate were alternately adjusted to maintain dissolved oxygen at 5-10%. After the cell OD562 increased to 20, the pH was increased to 6.9. When the residual sugar concentration decreased to 2 wt%, feeding was started, and at this time, the flow rate ratio of the supplementary solution A and the supplementary solution B was controlled to be 8: 1. After the cell OD562 increased to 30, the pH was increased to 7.1, and 1 mM lactose was added at one time (no addition was required for the strain IBBH-15). After the cell OD562 increased to 40, the pH was increased to 7.2. The residual sugar concentration was controlled at 2 wt%, and the fermentation broth was discharged after the feeding sugar was exhausted.

Table 11 Main Results of Example 10

| Strain | Treatment manner | Molasses | L-Leucine wt% | Sugar-to-Acid Conversion Rate % | Byproduct Acid wt% |
|---|---|---|---|---|---|
| IBCL Q-257 | Sucrase Hydrolysis Only | Beet Molasses | 6.25 | 31.25 | 0.09 |
| | Protease Hydrolysis Only | Beet Molasses | 6.29 | 30.45 | 0.07 |
| | Sucrase Hydrolysis Only | Cane Molasses | 6.15 | 30.75 | 0.09 |
| IBCL Q-257e | Sucrase Hydrolysis Only | Beet Molasses | 6.05 | 30.25 | 0.14 |
| | Protease Hydrolysis Only | Beet Molasses | 6.11 | 30.55 | 0.11 |
| | Sucrase Hydrolysis Only | Cane Molasses | 6.08 | 30.40 | 0.13 |
| IBBH 15 | Protease Hydrolysis Only | Cane Molasses | 5.15 | 25.75 | 0.16 |
| | Sucrase Hydrolysis Only | Beet molasses | 5.20 | 26.00 | 0.18 |
| | Protease hydrolysis only | Beet molasses | 5.18 | 25.90 | 0.16 |

As shown in Example 10, beet/cane molasses is hydrolyzed only with invertase or protease, followed by fed-batch of the corresponding molasses. The results show that partially hydrolyzed molasses leads to a decrease in leucine yield and sugar-to-acid conversation rate compared with Example 6, and that molasses without protease hydrolysis results in an increase in byproduct acids in the fermentation broth.

The present example proves that partially hydrolyzed molasses is still capable of improving leucine production, but the improvement ability is limited compared with Example 6.

**Comparative Example 1: Comparison of one-time addition process of molasses with fed-batch process**

1. The seed formulation and cultivation process are the same as those in Example 6.

2. The fermentation medium:

Initial fermentation medium in the standard fermentation tank: glucose 4 wt%, one-time addition of beet molasses hydrolysate 0.5-3.0 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A glucose 40-50 wt%.

3. The fermentation tank process (500 L):

One loop of each of the *Corynebacterium glutamicum* IBCLQ-257, IBCLQ-257e, and IBBH-15 was respectively inoculated into the primary seed medium in shake flasks, and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated into 50 L of the secondary seed tank medium at 0.2% (V/V). Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained. The secondary seed culture was inoculated into the 500 L fermentation tank at 10%, with an initial fermentation filling volume of 60%. Fermentation was carried out at 30°C with pH 6.7, a stirring speed of 200 rpm, an aeration rate of 60 L/min, and a tank pressure of 0.05 MPa. The stirring speed and aeration were alternately adjusted to maintain dissolved oxygen at 5-10%. After the cell OD562 increased to 20, the pH was increased to 6.9. When the residual sugar concentration decreased to 2 wt%, glucose feeding was started, and the residual sugar concentration was controlled at 2 wt%. After the cell OD562 increased to 30, the pH was increased to 7.1, and 1 mM lactose was added at one time (no addition was required for strain IBBH-15). After the cell OD562 increased to 40, the pH was increased to 7.2.

Table 12 Main Results of Comparative Example 1

| Strain | Molasses Added in One Time wt% | L-Leucine Yield wt% | Sugar-to-Acid Conversion Rate % | Byproduct Acid wt% |
|---|---|---|---|---|
| IBCL Q-257 | 2 | 5.45 | 26.55 | 0.09 |
| | 1 | 5.36 | 28.40 | 0.11 |
| | 3 | 5.22 | 28.60 | 0.15 |
| IBCL Q-257e | 0.5 | 4.23 | 21.15 | 0.13 |
| | 1.5 | 5.55 | 27.75 | 0.15 |
| | 2.5 | 5.24 | 26.20 | 0.21 |
| IBBH 15 | 4 | 4.28 | 21.40 | 0.14 |
| | 1 | 4.23 | 22.80 | 0.25 |
| | 2 | 4.56 | 20.80 | 0.26 |

The concentration of one-time addition of molasses described above is calculated as the total amount of molasses added divided by the initial fermentation volume.

As shown in the comparative example 1, one-time addition of molasses leads to a decrease in the acid production and the sugar-to-acid conversion rate. Compared with the fed-batch process, one-time addition of molasses results in either an excess or a deficiency of organic nitrogen sources in the fermentation broth, thereby causing abnormal acid production by the cells.

The comparative example 1 proves that the fed-batch process of molasses has obvious advantages over one-time addition.

**Comparative Example 2: Determination of initial added amount**

1. The seed formulation and cultivation process are the same as those in Example 6.

2. The fermentation medium:

Initial fermentation medium in the standard fermentation tank: glucose 4 wt%, beet molasses hydrolysate 0.3-0.6 wt% or 1.1-1.2 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%; supplementary solution B: beet molasses hydrolysate 40-50 wt%. During use, concentrations of the supplementary solution A and the supplementary solution B are required to be kept consistent.

3. The fermentation tank process (500 L):

One loop of each of the *Corynebacterium glutamicum* IBCLQ-257, IBCLQ-257e, and IBBH-15 was respectively inoculated into the primary seed medium in shake flasks, and cultivated at 30°C with reciprocal shaking at 85 rpm for 16 h to obtain 500 mL of the shake-flask seed culture. Maturity marker of the primary seed culture was OD562 of 8-12.

The shake-flask seed culture was inoculated at 0.2% (V/V) into 50 L of the secondary seed tank medium. Cultivation was carried out at 30°C with pH 6.8, and dissolved oxygen maintained not lower than 10%. After 16 h of cultivation, the maturity marker of the secondary seed culture was OD562 of 8-10, and the secondary seed culture was obtained. The stirring speed and aeration were alternately adjusted to maintain dissolved oxygen at 5-10%. After the cell OD562 increased to 20, the pH was increased to 6.9. When the residual sugar concentration decreased to 2 wt%, glucose feeding was started, and the residual sugar concentration was controlled at 2 wt%. After the cell OD562 increased to 30, the pH was increased to 7.1, and 1 mM lactose was added at one time (no addition was required for strain IBBH-15). After the cell OD562 increased to 40, the pH was increased to 7.2.

Table 13 Main Results of Comparative Example 2

| Str ain | Initial Concentrati on wt% | Total Concentration of Molasses wt% | Supplementary Solution A/Supplementary Solution AB | L-Leucin e wt% | Sugar-to-Acid Conversion Rate % | Byproduc t Acid wt% |
|---|---|---|---|---|---|---|
| IB CL | 0.8 | 4.0 | 5:1 | 5.90 | 29.50 | 0.12 |
| | 0.3 | 2.5 | 7:1 | 5.83 | 29.20 | 0.16 |
| Q-25 7 | 1.2 | 2.3 | 13:1 | 5.52 | 27.60 | 0.12 |
| IB CL Q-25 7e | 0.8 | 4 | 5:1 | 5.35 | 26.75 | 0.21 |
| | 0.3 | 2.5 | 7:1 | 5.24 | 26.20 | 0.22 |
| | 1.2 | 2.3 | 13:1 | 5.14 | 25.70 | 0.19 |
| IB BH - 15 | 0.6 | 4.6 | 4:1 | 4.56 | 22.80 | 0.28 |
| | 1.1 | 2.0 | 12:1 | 4.55 | 22.75 | 0.25 |
| | 1.2 | 3.5 | 7:1 | 4.11 | 20.55 | 0.24 |

The initial concentration refers to the actual concentration of molasses in the initial medium. The total concentration of molasses is calculated as the total amount of molasses divided by the final fermentation broth volume.

As shown in the Comparative Example 2, an excessively high or low initial concentration of molasses, excessively high or low feeding rate of molasses, or excessively high or low total amount of molasses all cause a decrease in the acid production and the sugar-to-acid conversion rate. Compared with the Example 1 and Example 2, the Comparative Example 2 illustrates several extreme addition conditions of molasses, demonstrating the importance of controlling the initial added amount of molasses, controlling the supplementary feeding ratio and rate, and controlling the total amount of molasses.

**Comparative Example 3: Determination of achieving the effect of increasing acid production by hydrolyzing molasses**

1. The seed formulation and cultivation process are the same as those in Example 6.

2. The fermentation medium:

Initial fermentation medium in the standard fermentation tank: glucose 4 wt%, raw beet/cane molasses 0.8 wt%, ammonium sulfate 0.4 wt%, potassium dihydrogen phosphate 0.11 wt%, magnesium sulfate 0.05 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%; supplementary solution B: molasses hydrolysate 40-50 wt%. During use, concentrations of the supplementary solution A and the supplementary solution B are required to be kept consistent.

3. The fermentation tank process (500 L):

One loop of each of the *Corynebacterium glutamicum* IBCLQ-257, IBCLQ-257e, and IBBH-15 was respectively inoculated into the primary seed medium in shake flasks, and cultivated at 30°C with reciprocal shaking at 85 rpm for 16 h to obtain 500 mL of the shake-flask seed culture. Maturity marker of the primary seed culture was OD562 of 8-12.

The secondary seed culture was inoculated into the 500-L fermentation tank at 10%, with a fermentation volume of 300 L. Fermentation was carried out at 30°C with pH 6.7, the stirring speed of 200 rpm, an aeration rate of 60 L/min, and a tank pressure of 0.05 MPa. The stirring speed and aeration were alternately adjusted to maintain dissolved oxygen at 5-10%. After the cell OD562 increased to 20, the pH was increased to 6.9. When the residual sugar concentration decreased to 2 wt%, feeding was started, and at this time, the flow rate ratio of the supplementary solution A and the supplementary solution B was controlled to be 8:1. After the cell OD562 increased to 30, the pH was increased to 7.1, and 1 mM lactose was added at one time (no addition was required for the strain IBBH-15). After the cell OD562 increased to 40, the pH was increased to 7.2. The residual sugar concentration was controlled at 2 wt%, and the fermentation broth was discharged after the feeding sugar was exhausted.

Table 14 Main Results of Comparative Example 3

| Strain | Beet Molasses | Cane Molasses | L-Leucine wt% | Sugar-to-Acid Conversion Rate % | Byproduct Acid wt% |
|---|---|---|---|---|---|
| IBCLQ-257 | 100% | 0 | 5.92 | 29.60 | 0.15 |
| | 0 | 100% | 5.85 | 29.25 | 0.16 |
| | 50% | 50% | 5.82 | 29.10 | 0.18 |
| IBCLQ-257e | 100% | 0 | 5.55 | 27.75 | 0.25 |
| | 0 | 100% | 5.48 | 27.40 | 0.24 |
| | 50% | 50% | 5.44 | 27.20 | 0.30 |
| IBBH-15 | 100% | 0 | 4.76 | 23.80 | 0.28 |
| | 0 | 100% | 4.42 | 22.10 | 0.29 |
| | 50% | 50% | 4.65 | 23.25 | 0.30 |

As shown in Comparative Example 3, when unhydrolyzed molasses is used for L-leucine fermentation, the acid production and the sugar-to-acid conversion rate do not reach the effect achieved by hydrolyzed molasses. The comparative example indicates that molasses after hydrolysis is advantageous to L-leucine fermentation. The sucrose in hydrolyzed molasses is hydrolyzed into the glucose and the fructose, and crude protein is decomposed, which is more beneficial to cell metabolism, whereas unhydrolyzed molasses does not achieve the effect.

**Comparative Example 4: Determination of the process advantages**

1. A composition of the seed medium:

Primary seed medium: glucose 3 wt%, corn steep liquor 4 wt%, ammonium sulfate 0.5 wt%, potassium dihydrogen phosphate 0.1 wt%, magnesium sulfate 0.05 wt%, calcium carbonate 1 wt%, pH 6.7-7.2; dispensed at 500 mL/5000 mL; sterilization condition 121°C/20 min.

Secondary seed tank medium: glucose 3 wt%, corn steep liquor 4 wt%, ammonium sulfate 0.5 wt%, potassium dihydrogen phosphate 0.1 wt%, magnesium sulfate 0.05 wt%, pH 6.8-7.2; sterilization condition 121°C/20 min.

2. The fermentation medium:

Initial fermentation medium in the standard fermentation tank: glucose 10 wt%, corn steep liquor 1 wt%, ammonium sulfate 0.5 wt%, potassium dihydrogen phosphate 0.11 wt%, magnesium sulfate 0.25 wt%, ferrous sulfate 0.001 wt%, manganese sulfate 0.001 wt%, biotin 0.00001 wt%, vitamin B1 0.00003 wt%, L-methionine 0.01 wt%, L-isoleucine 0.01 wt%; natural pH; sterilization condition 121°C/20 min.

Supplementary solution: supplementary solution A: glucose 40-50 wt%, magnesium sulfate 0.05 wt%.

3. The fermentation tank process (500 L):

One loop of each of the *Corynebacterium glutamicum* IBCLQ-257, IBCLQ-257e, and IBBH-15 was respectively inoculated into the primary seed medium in shake flasks, and cultivated at 30°C with reciprocal shaking at 85 rpm for 16 h to obtain 500 mL of the shake-flask seed culture. Maturity marker of the primary seed culture was OD562 of 8-12.

The secondary seed culture was inoculated at 10% into the 500 L fermentation tank, with a fermentation volume of 300 L. Fermentation was carried out at 30°C with pH 6.7, the stirring speed of 200 rpm, an aeration rate of 60 L/min, and a tank pressure of 0.05 MPa. The stirring speed and aeration rate were alternately adjusted to maintain dissolved oxygen at 5-10%. After the cell OD562 increased to 20, the pH was increased to 6.9. When the residual sugar concentration decreased to 2 wt%, feeding was started. After the cell OD562 increased to 30, the pH was increased to 7.1, and 1 mM lactose was added at one time (no addition was required for strain IBBH-15). After the cell OD562 increased to 40, the pH was increased to 7.2. After 48 h, 1 mM lactose was added. The residual sugar concentration was controlled at 2-3 wt%, and after the supplementary sugar was consumed, fermentation was terminated and the broth was discharged.

Table 15 Main Results of Comparative Example 4

| Strain | L-Leucine Yield wt% | Sugar-to-Acid Conversion Rate % | Byproduct Acid wt% |
|---|---|---|---|
| IBCLQ-257 | 6.01 | 27.8 | 0.12 |
| IBCLQ-257e | 5.76 | 26.6 | 0.15 |
| IBBH-15 | 4.95 | 24.5 | 0.29 |

As shown in Comparative Example 4, Comparative Example uses corn steep liquor for L-leucine fermentation. A comparison with Example 1 shows that using hydrolyzed molasses for L-leucine fermentation results in higher acid production, a higher sugar-to-acid conversion rate, and fewer byproduct acids. In addition, molasses contains fewer impurities than corn steep liquor, has a lighter color, and is more favorable for subsequent separation and purification.

**Example 11: Effect of sRNAs25 expression in the genome of the L-leucine-producing strain on acid production**

The one loop of slant bacterial lawn f from each of L-leucine-producing strain IBBH-15, the genetically engineered strain IBCLQ-257e, and IBCLQ-257 was respectively inoculated into four 3 L shake flasks, cultivated at 80 rpm and 30°C until the cell OD562 reached approximately 15, and the shake-flask seed culture was obtained for later use. The shake-flask seed culture was inoculated into the 30 L fermentation tank fermentation medium at an inoculation amount of 10% (V/V). At 48 h, 1 mM lactose was added to induce plasmid expression. The initial temperature was 30°C, and the initial aeration rate was 0.8 L/min. By controlling the stirring speed and aeration rate, the dissolved oxygen was maintained at 10-20% during the first 24 h of the fermentation process and at 5-15% after 24 h. The pH of the fermentation process was controlled by automatically feeding ammonia water. The initial pH was controlled at 6.7. After the cell OD562 increased to 20, the pH was increased to 6.9; after the cell OD562 increased to 25, the pH was increased to 7.0; after the cell OD562 increased to 36, the pH was increased to 7.2. During the fermentation process, the residual sugar concentration was controlled at 20-30 g/L.

Table 16 Main Results of Example 11

| Strain | Yield g/L | Sugar-to-Acid Conversion Rate % | Fermentation Period h | Byproduct Acid g/L |
|---|---|---|---|---|
| IBBH-15 | 49.8±2.13 | 24.6 | 72 | 2.58±0.35 |
| IBCLQ-257 | 60.02 ±2.01 | 27.7 | 79 | 0.96±0.15 |

(continued)

| Strain | Yield g/L | Sugar-to-Acid Conversion Rate % | Fermentation Period h | Byproduct Acid g/L |
|---|---|---|---|---|
| IBCLQ-257e | 57.66 ±3.23 | 26.6 | 51 | 1.22±0.25 |

The data from Table 16 show that the genetically engineered strain IBCLQ-257e, in which the non-coding sRNA s25 is expressed on the genome, shows the acid production capacity and the byproduct acid proportion similar to those of the strain IBCLQ-257, in which sRNA s25 is overexpressed in the previous example. However, the fermentation cycle is shortened by 15.3% compared with the original strain, greatly reducing labor and energy consumption. In addition, there is no antibiotic risk in the genome, and there are obvious overall advantages.

The initial fermentation medium of the fermentation tank: glucose 100 g/L, ammonium sulfate 5 g/L, corn steep liquor 10 mL/L, potassium dihydrogen phosphate 5 g/L, magnesium sulfate 2.5 g/L, ferrous sulfate 0.01 g/L, manganese sulfate 0.01 g/L, biotin 100 $\mu$g/L, vitamin B1 300 $\mu$g/L, L-methionine 0.1 g/L, L-isoleucine 0.1 g/L, L-glutamate 0.1 g/L, vegetable oil 1 mL/L, pH 7.0-7.2.

The supplementary sugar solution: glucose 400 g/L, magnesium sulfate 0.5 g/L.

**Example 12: Preferred example with beet molasses hydrolysate, fed-batch, and optimal ratio A/B of 5/1**

1. The composition of the seed medium:

The primary seed medium: glucose 3 wt%, corn steep powder 1.0 wt%, ammonium sulfate 0.5 wt%, KH$_2$PO$_4$ 0.1 wt%, MgSO$_4$·7H$_2$O 0.05 wt%, calcium carbonate 1.0 wt%, pH 6.7-7.2; dispensed at 500 mL/5000 mL; sterilization condition 121°C/20 min.

Secondary seed tank medium: glucose 5 wt%, beet molasses hydrolysate 3.0 wt%, ammonium sulfate 0.5 wt%, KH$_2$PO$_4$ 0.1 wt%, MgSO$_4$•7H$_2$O 0.05 wt%, pH 6.8-7.2; sterilization condition 121°C/20 min.

2. The fermentation medium:

Initial fermentation medium in the standard fermentation tank: glucose 10 wt%, beet molasses hydrolysate 2 wt%, ammonium sulfate 1.0 wt%, potassium dihydrogen phosphate 0.1 wt%, magnesium sulfate 0.1 wt%.

Supplementary solutions: the supplementary solution A: glucose 40-50 wt%, potassium dihydrogen phosphate 0.1 wt%, magnesium sulfate 0.05 wt%; the supplementary solution B: beet molasses hydrolysate 40-50 wt%; the supplementary solution C: ammonium sulfate solution 10-12%. During use, the glucose concentration and the molasses concentration need to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in the shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration rate and stirring speed to maintain dissolved oxygen at approximately 10%. When the OD562 of the strain in the fermentation broth reached approximately 40, the pH was adjusted to approximately 7.0, and 1 mM lactose was added at one time (no addition was required for the strain IBCL-1). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%, and the feeding ratio of the supplementary solution A and the supplementary solution B was controlled at 5/1. Meanwhile, feeding the inorganic salt supplementary solution was continued to control the osmolality of the fermentation broth at approximately 900 mosm/L. The fermentation was terminated when the glucose was completely consumed.

Table 17 Main Results of Example 12

| Strain | L-Isoleucine wt% | Sugar-to-Acid Conversion Rate % | Byproduct Acid wt% |
|---|---|---|---|
| IBCIL-253 | 7.25 | 41.9 | 0.06 |
| IBCIL-253k | 7.03 | 40.64 | 0.09 |
| IBCL1 | 6.58 | 38.0 | 0.12 |

As shown in Example 12, the core of the present disclosure is that the beet molasses is hydrolyzed using invertase and protease to obtain the beet molasses hydrolysate. Subsequently, during L-isoleucine fermentation, the glucose solution and the beet molasses hydrolysate are fed at a certain ratio to control the residual sugar concentration in the fermentation broth. In this way, not only is the residual sugar controlled, but excessively rapid cell growth is also prevented through feeding an organic nitrogen source. In the hydrolyzed beet molasses, sucrose is completely hydrolyzed into the glucose and the fructose, which are more easily utilized by cells. The protease hydrolyzes macromolecular proteins so that the nitrogen source is fully utilized, thereby achieving the purpose that both carbon source and nitrogen source of the molasses are completely utilized.

In the fermentation process, the acid production refers to the actual concentration of L-isoleucine in the fermentation broth.

The calculation formula of the sugar-to-acid conversion rate is as follows:

Sugar-to-acid conversion rate = (actual concentration of L-isoleucine $\times$ actual fermentation volume)/(total actual consumption amount of glucose $\times$ 100%).

Meanwhile, the byproduct acids refer to the sum of valine, lysine, alanine, and glutamic acid in the fermentation broth. The sugar-to-acid conversion rate and the byproduct acids are jointly used to evaluate the efficiency of the fermentation process and the composition of the products.

In summary, the *Corynebacterium glutamicum* IBCIL-253, IBCL-1, and IBCIL-253k show significant advantages in L-isoleucine yield, the sugar-to-acid conversion rate, and the byproduct acids in the process of Example 1.

**Example 13: Determination of initial added amount range and the ratio A/B**

1. The seed process is consistent with Example 12.

2. The fermentation medium:

The initial fermentation medium in the standard fermentation tank: glucose 10 wt%, beet molasses hydrolysate 1.5-2.5 wt%, ammonium sulfate 1.0 wt%, potassium dihydrogen phosphate 0.1 wt%, and magnesium sulfate 0.1 wt%.

The supplementary solutions: the supplementary solution A: glucose 40-50 wt%, potassium dihydrogen phosphate 0.1 wt%, and magnesium sulfate 0.05 wt%; the supplementary solution B: beet molasses hydrolysate 40-50 wt%; the supplementary solution C: ammonium sulfate solution 10-12%. During use, the concentrations of the glucose and the molasses need to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 $\times$ 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 $\times$ 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration and stirring speed to maintain dissolved oxygen at approximately 10%. When the OD562 of the strain in the fermentation broth grew to approximately 40, the pH was adjusted to approximately 7.0, and 1 mM lactose was added at one time (no addition was required for the strain IBCL-1). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar at approximately 2.0 wt%, and the feeding ratio of the supplementary solution A and the supplementary solution B was controlled at 4/1-7/1. Meanwhile, feeding the inorganic salt supplementary solution was continued to control the osmolality of the fermentation broth at approximately 900 mosm/L. The fermentation was terminated when the glucose was completely consumed.

Table 18 Main Results of Example 13

| Strai n | Initial Concentration wt% | A/ B | L-Isoleucine Yield wt% | Sugar-to-Acid Conversion Rate % | Byproduct Acid wt% |
|---|---|---|---|---|---|
| IBCI L-253 | 1.5 | 4: 1 | 7.02 | 40.6 | 0.06 |
| | 1.8 | 6: 1 | 7.12 | 41.2 | 0.07 |
| | 2.5 | 7: 1 | 6.89 | 39.8 | 0.09 |
| IBCI L-253k | 1.5 | 6: 1 | 6.85 | 38.9 | 0.12 |
| | 1.8 | 7: 1 | 6.79 | 38.6 | 0.13 |
| | 2.5 | 4: 1 | 6.95 | 39.5 | 0.12 |
| IBCL -1 | 1.6 | 6: 1 | 6.32 | 36.5 | 0.11 |
| | 2 | 4: 1 | 6.41 | 37.1 | 0.15 |
| | 2.5 | 7: 1 | 6.33 | 36.6 | 0.11 |

As shown in Example 13, controlling the initial added amount of the beet molasses hydrolysate at 1.5-2.5 wt% and maintaining a feeding ratio of supplementary solution A to supplementary solution B in a range of 4/1-7/1 yield favorable results, which are slightly lower than those of Example 1. Example 13 demonstrates that the initial added amount of the molasses hydrolysate and the ratio of the supplementary solution A to the supplementary solution B during supplementary feeding are both critical to improving the acid production and to controlling the byproduct acids in L-isoleucine fermentation.

**Example 14: Determination that the molasses hydrolysate process is applicable to beet molasses and cane molasses**

1. The seed process is consistent with Example 12.

2. The fermentation medium:

The initial fermentation medium in the standard fermentation tank: glucose 10 wt%, a mixture of beet molasses hydrolysate and cane molasses hydrolysate at a certain ratio 2 wt%, ammonium sulfate 1.0 wt%, potassium dihydrogen phosphate 0.1 wt%, magnesium sulfate 0.1 wt%.

Supplementary solutions: supplementary solution A: glucose 40-50 wt%, potassium dihydrogen phosphate 0.1 wt%, magnesium sulfate 0.05 wt%; supplementary solution B: the mixture of beet molasses hydrolysate and the cane molasses hydrolysate 40-50 wt%; supplementary solution C: ammonium sulfate solution 10-12%. During use, the glucose concentration and the molasses concentration need to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 $\times$ 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 $\times$ 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration and stirring speed to maintain dissolved oxygen at approximately 10%. When the $OD_{562}$ of the strain in the fermentation broth grew to approximately 40, the pH was adjusted to approximately 7.0, and 1 mM lactose was added at one time (no addition was required for the strain IBCL-1). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%, and the feeding ratio of the supplementary solution A and the supplementary solution B was controlled at 5/1. Meanwhile, feeding the inorganic salt supplementary solution was continued to control the osmolality of the fermentation broth at approximately 900 mosm/L. The fermentation was terminated when the glucose was completely consumed.

Table 19 Main Results of Example 14

| Strain | Cane Molasses Hydrolysate Percentage % | Beet Molasses Hydrolysate Percentage % | L-Isoleucin e wt% | Sugar-to-Acid Conversion Rate % | Byproduct Acid wt% |
|---|---|---|---|---|---|
| IBCI-L-253 | 100 | 0 | 6.95 | 40.2 | 0.07 |
| | 50 | 50 | 6.92 | 40.0 | 0.07 |
| | 20 | 80 | 7.01 | 40.5 | 0.08 |
| IB-CI-L-253k | 90 | 10 | 6.74 | 38.3 | 0.13 |
| | 50 | 50 | 6.78 | 38.5 | 0.12 |
| | 20 | 80 | 6.81 | 38.7 | 0.11 |
| IBCL-1 | 100 | 0 | 6.46 | 37.3 | 0.15 |
| | 80 | 20 | 6.35 | 36.7 | 0.16 |
| | 10 | 90 | 6.52 | 37.7 | 0.15 |

The data from Table 19 show that after the cane molasses is hydrolyzed using sucrase and protease and then mixed with the beet molasses hydrolysate at a certain ratio, the ideal effect is also achieved. However, when the cane molasses hydrolysate is used alone, the effect is slightly lower than that of the beet molasses in Example 12.

Example 14 demonstrates that the cane molasses hydrolysate can be applied as an organic nitrogen source in an L-isoleucine process.

**Example 15: Determination of partially hydrolyzed molasses being used for fermentation**

1. The seed process is consistent with Example 12.

2. The fermentation medium:

Initial fermentation medium in the standard fermentation tank: glucose 10 wt%, partially hydrolyzed beet/cane molasses 2 wt%, ammonium sulfate 1.0 wt%, potassium dihydrogen phosphate 0.1 wt%, magnesium sulfate 0.1 wt%.

Supplementary solutions: supplementary solution A: glucose 40-50 wt%, potassium dihydrogen phosphate 0.1 wt%, magnesium sulfate 0.05 wt%; supplementary solution B: partially hydrolyzed beet or the cane molasses hydrolysate 40-50 wt%; supplementary solution C: ammonium sulfate solution 10-12%. During use, the concentrations of the glucose and the molasses are required to remain consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the $OD_{562}$ of 0.6-0.7 × 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration and stirring speed to maintain dissolved oxygen at approximately 10%. When the OD562 of the strain in the fermentation broth grew to approximately 40, the pH was adjusted to approximately 7.0, and 1 mM lactose was added at one time (no addition was required for the strain IBCL-1). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%, and the feeding ratio of the supplementary solution A and the supplementary solution B was controlled at 5/1. Meanwhile, feeding the inorganic salt supplementary solution was continued to control the osmolality of the fermentation broth at approximately 900 mosm/L. The fermentation was terminated when the glucose was completely consumed.

Table 20 Main Results of Example 15

| Strain | Treatment Manner | Molasses | L-Isoleucine wt% | Sugar-to-Acid Conversion Rate % | Byproduct Acid wt% |
|---|---|---|---|---|---|
| IBCI L-253 | Sucrase hydrolysis only | Beet molasses | 6.74 | 39.0 | 0.09 |
| | Protease hydrolysis only | Beet molasses | 6.79 | 39.2 | 0.07 |
| | Sucrase hydrolysis only | Cane molasses | 6.75 | 39.0 | 0.09 |
| IBCI L-253k | Sucrase hydrolysis only | Beet molasses | 6.64 | 37.7 | 0.15 |
| | Protease hydrolysis only | Beet molasses | 6.69 | 38.0 | 0.11 |
| | Sucrase hydrolysis only | Cane molasses | 6.65 | 37.8 | 0.16 |
| IBCL -1 | Protease hydrolysis only | Cane molasses | 6.22 | 36.0 | 0.15 |
| | Sucrase hydrolysis only | Beet molasses | 6.24 | 36.1 | 0.16 |
| | Protease hydrolysis only | Beet molasses | 6.28 | 36.3 | 0.16 |

As shown in Example 15, two types of molasses are respectively subjected to invertase hydrolysis and protease hydrolysis, and after hydrolysates are obtained, verification of L-isoleucine fermentation is carried out. Example 15 indicates that partially hydrolyzed molasses can still be applied in the fermentation process, but the fermentation result is lower compared with that of complete hydrolysis.

**Example 16: Determination of a range of a total amount of used molasses**

1. The seed process is consistent with Example 12.

2. The fermentation medium:

Initial fermentation medium in the standard fermentation tank: glucose 10 wt%, beet molasses hydrolysate 2 wt%, ammonium sulfate 1.0 wt%, potassium dihydrogen phosphate 0.1 wt%, and magnesium sulfate 0.1 wt%.

Supplementary solutions: supplementary solution A: glucose 40-50 wt%, potassium dihydrogen phosphate 0.1 wt%, and magnesium sulfate 0.05 wt%; supplementary solution B: beet molasses hydrolysate 40-50 wt%; supplementary solution C: ammonium sulfate solution 10-12%. During use, concentrations of glucose and molasses are required to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration and stirring speed to maintain dissolved oxygen at approximately 10%. When the OD562 of the strain in the fermentation broth grew to approximately 40, the pH was adjusted to approximately 7.0, and 1 mM lactose was added at one time (no addition was required for the strain IBCL-1). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%, and the feeding ratio of the supplementary solution A and the supplementary solution B was controlled at 4/1. Meanwhile, feeding the inorganic salt supplementary solution was continued to control the osmolality of the fermentation broth at approximately 900 mosm/L. The fermentation was terminated when the glucose was completely consumed.

Table 21 Main Results of Example 16

| Strain | Total Concentration of Molasses wt% | L-isoleucine wt% | Sugar-to-acid conversion rate % | Byproduct acid wt% |
|---|---|---|---|---|
| IB-CI-L-253 | 3.8 | 6.83 | 39.5 | 0.08 |
| | 3.5 | 6.92 | 40.0 | 0.1 |
| | 4.5 | 6.81 | 39.4 | 0.08 |
| | 4.2 | 6.86 | 39.7 | 0.06 |
| IB-CI-L-253k | 3.5 | 6.77 | 39.1 | 0.11 |
| | 4 | 6.76 | 39.1 | 0.12 |
| | 4.5 | 6.66 | 38.5 | 0.14 |
| | 3.9 | 6.75 | 39.0 | 0.17 |
| IBCL-1 | 4.2 | 6.32 | 36.5 | 0.14 |
| | 3.9 | 6.27 | 36.2 | 0.16 |
| | 3.5 | 6.26 | 36.2 | 0.15 |
| | 4.5 | 6.24 | 36.1 | 0.17 |

The total concentration of molasses refers to a total amount of molasses divided by the final fermentation volume.

As shown in Example 16, optimization verification of the total amount of molasses is respectively carried out, and results indicate that when the total concentration of the molasses hydrolysate is in a range of 3.5-4.5 wt%, an ideal result is achieved. Example 16 indicates that the amount of the molasses hydrolysate has a certain requirement, which is not too low or too high.

**Example 17: Effect of sRNAs25 expression in the L-isoleucine-producing strain on the acid production**

The one loop of slant bacterial lawn from each the L-isoleucine-producing strain IBCL-1 and genetically engineered strains IBCIL-253k and IBCIL-253 was respectively inoculated into two into 3 L shake flasks, followed by oscillatory cultivation at 30°C and 90 rpm for 16 h until the OD562 reached about 15 to obtain the shake-flask seed culture for later use. The shake-flask seed culture was inoculated into the fermentation medium in a 30 L fermentation tank at an inoculation amount of 10% (V/V), and at 18 h, 1 mM lactose was added to induce plasmid expression. An initial temperature was 30°C and pH was 6.8, and during the fermentation process, the dissolved oxygen level was controlled at about 10% by continuously adjusting aeration rate and the stirring speed. At 48 h, 1 mM lactose was added to induce plasmid expression. When the cell OD562 in the fermentation broth grew to about 40, the pH was adjusted to about 7.0; when the residual sugar concentration decreased to 6%, feeding of the inorganic salt supplementary solution was performed to control the fermentation broth osmolality at about 700 mosm/L; when the residual sugar concentration was lower than 1%, feeding of the supplementary glucose solution was performed to maintain the residual sugar concentration at about 1%, while the inorganic salt supplementary solution was continuously fed to control the fermentation broth osmolality at about 900 mosm/L, and the fermentation was completed after the sugar was depleted at about 30 h. The fermentation yield is shown in Table 22.

Table 22 Main Results of Example 17

| Strain | Yield g/L | Sugar-to-acid conversion rate | Fermentation period | Byproduct acid g/L |
|---|---|---|---|---|
| IBCL-1 | 61.7±1.40 | 36.3% | 60 | 1.74±0.40 |
| IBCIL-253 | 66.4±3.20 | 38.2% | 65 | 0.84±0.12 |
| IBCIL-253k | 65.31±3.11 | 37.6% | 54 | 0.96±0.91 |

Table 22 indicates that the genetically engineered strain IBCIL-253k expressing the non-coding sRNA s25 on the genome has the acid production capacity and the byproduct acid ratio similar to those of strain IBCIL-253 overexpressing sRNA s25 in the previous example, but the fermentation period is shortened by 10% compared with that of an original strain, which greatly reduces labor energy consumption, and there is no antibiotic risk in the genome, thereby having obvious overall advantages.

The seed medium: glucose 30 g/L, corn steep powder 10 g/L, ammonium sulfate 5 g/L, potassium dihydrogen phosphate 1 g/L, magnesium sulfate 0.5 g/L, calcium carbonate 10 g/L, and pH 6.7-7.2; dispensed at 500 mL/5000 mL; sterilization condition 121°C/20 min.

The fermentation medium: glucose 100 g/L, corn steep powder 8 g/L, ammonium sulfate 5 g/L, potassium dihydrogen phosphate 1 g/L, and magnesium sulfate 0.5 g/L.

The supplementary glucose solution: glucose 400 g/L, potassium dihydrogen phosphate 1 g/L, and magnesium sulfate 0.5 g/L.

The inorganic salt supplementary solution: ammonium sulfate 100 g/L.

**Comparative Example 5: Comparison between a process of one-time addition of molasses and a fed-batch process**

1. The seed process is consistent with Example 12.

2. The fermentation medium:

The initial fermentation medium in the standard fermentation tank: glucose 10 wt%, beet molasses hydrolysate 2.5-4.5 wt%, ammonium sulfate 1.0 wt%, potassium dihydrogen phosphate 0.1 wt%, and magnesium sulfate 0.1 wt%.

Supplementary solutions: supplementary solution A: glucose 40-50 wt%, potassium dihydrogen phosphate 0.1 wt%, and magnesium sulfate 0.05 wt%; supplementary solution C: ammonium sulfate solution 10-12%. During use, concentrations of glucose and molasses are required to be kept consistent.

3. Fermentation tank process (500 L).

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration rate and stirring speed to maintain dissolved oxygen at approximately 10%. When the OD562 of the strain in the fermentation broth grew to approximately 40, the pH was adjusted to approximately 7.0, and 1 mM lactose was added at one time (the strain IBCL-1 does not require addition). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%. Meanwhile, feeding the inorganic salt supplementary solution was continued to control the osmolality of the fermentation broth at approximately 900 mosm/L. The fermentation was terminated when the glucose was completely consumed.

Table 23 Main Results of Comparative Example 5

| Strai n | One-time addition of molasses wt% | L-isoleucine yield wt% | Sugar-to-acid conversion rate % | Byproduct acid wt% |
|---|---|---|---|---|
| IBCI L-253 | 3.5 | 6.45 | 37.3 | 0.09 |
| | 4.5 | 6.38 | 36.9 | 0.11 |
| | 2.5 | 6.44 | 37.2 | 0.15 |
| IBCI L-253k | 3.6 | 6.34 | 36.0 | 0.17 |
| | 4.2 | 6.24 | 35.5 | 0.18 |
| | 2.9 | 6.25 | 35.5 | 0.17 |
| IBCL -1 | 3.4 | 6.14 | 35.5 | 0.14 |
| | 4.3 | 5.98 | 34.6 | 0.21 |
| | 3 | 6.01 | 34.7 | 0.22 |

The concentration of the molasses hydrolysate in the one-time addition refers to an amount of the molasses hydrolysate added based on an initial fermentation volume.

Comparative Example 5 indicates that the acid production results of one-time addition of molasses are lower compared with those of the examples, mainly because the one-time addition of the organic nitrogen source at the excessively high level causes cell growth to be too fast, which results in more sugar being wasted for cell propagation, thereby resulting in the acid production not reaching the expected effect; therefore, the fed-batch process is more suitable for L-isoleucine fermentation.

**Comparative Example 6: Determination of an initial added amount**

1. The seed process is consistent with Example 11.

2. The fermentation mediums:

The initial fermentation medium in the standard fermentation tank: glucose 10 wt%, beet molasses hydrolysate 1 or 3 wt%, ammonium sulfate 1.0 wt%, potassium dihydrogen phosphate 0.1 wt%, and magnesium sulfate 0.1 wt%.

Supplementary solutions: supplementary solution A: glucose 40-50 wt%, potassium dihydrogen phosphate 0.1 wt%, and magnesium sulfate 0.05 wt%; supplementary solution B: beet molasses hydrolysate 40-50 wt%; supplementary solution C: ammonium sulfate solution 10-12%. During use, concentrations of the glucose and the molasses are required to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.

Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.

Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration rate and stirring speed to maintain dissolved oxygen at approximately 10%. When the OD562 of the strain in the fermentation broth grew to approximately 40, the pH was adjusted to approximately 7.0, and 1 mM lactose was added at one time (no addition was required for the strain IBCL-1). When the residual sugar in the fermentation broth decreased to 6 wt%, feeding the ammonium sulfate supplementary solution was started to control the osmolality of the fermentation broth at approximately 700 mosm/L. (2) When the residual sugar was lower than 2 wt%, feeding was performed to maintain the residual sugar concentration at approximately 2.0 wt%, and the feeding ratio of the supplementary solution A and the supplementary solution B was controlled at 5/1. Meanwhile, feeding the inorganic salt supplementary solution was continued to control the osmolality of the fermentation broth at approximately 900 mosm/L. The fermentation was terminated when the glucose was completely consumed.

Table 24 Main Results of Comparative Example 6

| Strai n | Initial Concentration (wt%) | L-Isoleucine (wt%) | Sugar-to-Acid Conversion Rate (%) | Byproduct Acid (wt%) |
|---|---|---|---|---|
| IBCI L-253 | 1 | 6.34 | 36.6 | 0.14 |
| | 3 | 6.33 | 36.6 | 0.16 |
| IBCI L-253K | 1 | 6.24 | 35.5 | 0.21 |
| | 3 | 6.22 | 35.3 | 0.22 |
| IBCL -1 | 3 | 5.89 | 34.0 | 0.28 |
| | 1 | 6.11 | 35.3 | 0.29 |

As shown in Comparative Example 6, verification of different initial added amounts of the molasses hydrolysate is respectively carried out, and Comparative Example 6 indicates that during addition of the molasses hydrolysate, an initial added amount is particularly important, which is required to be controlled in a range of 1.5-2.5 wt% as described in the examples.

**Comparative Example 7: Determination of a feeding ratio of the supplementary solution A and the supplementary solution B**

1. The seed process is consistent with Example 11.

2. The fermentation medium:

Initial fermentation medium in the standard fermentation tank: glucose 10 wt%, beet molasses hydrolysate 1.8-2.2 wt%, ammonium sulfate 1.0 wt%, potassium dihydrogen phosphate 0.1 wt%, and magnesium sulfate 0.1 wt%.
Supplementary solutions: supplementary solution A: glucose 40-50 wt%, potassium dihydrogen phosphate 0.1 wt%, and magnesium sulfate 0.05 wt%; supplementary solution B: beet molasses hydrolysate 40-50 wt%; supplementary solution C: ammonium sulfate solution 10-12%. During use, concentrations of glucose and molasses are required to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.
Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.
Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration rate and stirring speed to maintain dissolved oxygen at approximately 10%. When the OD562 of the strain in the fermentation broth grew to approximately 40, the pH was adjusted to approximately 7.0, and 1 mM lactose was added at one time (no addition was required for the strain IBCL-1). When the residual sugar concentration in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%, and the feeding ratio of the supplementary solution A and the supplementary solution B was controlled at 3/1 or 8/1. Meanwhile, feeding the inorganic salt supplementary solution was continued to control the osmolality of the fermentation broth at approximately 900 mosm/L. The fermentation was terminated when the glucose was completely consumed.

Table 25 Main Results of Comparative Example 7

| Strain | Initial Concentration (wt%) | L-Isoleucine (wt%) | Sugar-to-Acid Conversion Rate (%) | Byproduct Acid (wt%) |
|---|---|---|---|---|
| IBCIL-253 | 1.8 | 6.25 | 36.1 | 0.11 |
| | 2 | 6.59 | 38.1 | 0.18 |
| IBCIL-253K | 2.1 | 6.22 | 35.3 | 0.21 |
| | 2 | 6.3 | 35.8 | 0.22 |
| IBCL-1 | 2.2 | 5.95 | 34.4 | 0.22 |
| | 2 | 6.07 | 35.1 | 0.24 |

As shown in Comparative Example 7, verification of a feeding ratio of the supplementary solution A and the supplementary solution B is respectively carried out, and results indicate that the feeding ratio is required to be controlled in a range of 4:1-7:1 as described in the examples, and too high or too low does not meet requirements.

**Comparative Example 8: Determination that molasses must be hydrolyzed to achieve an effect of improving acid production**

1. The seed process is consistent with Example 11.

2. The fermentation medium:

The initial fermentation medium in the standard fermentation tank: glucose 10 wt%, beet/cane molasses 2 wt%, ammonium sulfate 1.0 wt%, potassium dihydrogen phosphate 0.1 wt%, and magnesium sulfate 0.1 wt%.
Supplementary solutions: supplementary solution A: glucose 40-50 wt%, potassium dihydrogen phosphate 0.1 wt%, and magnesium sulfate 0.05 wt%; supplementary solution B: beet/cane molasses 40-50 wt%; supplementary solution C: ammonium sulfate solution 10-12%. During use, concentrations of glucose and molasses are required to be kept consistent.

3. Fermentation tank process (500 L):

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.
Step 2: Inoculating the shake-flask seed culture into 50 L of the secondary seed tank medium at a ratio of 1% (V/V), followed by fermentation cultivation at 30°C, 200 rpm, and an aeration ratio of 0.3 VVM, with pH 6.8 and dissolved oxygen not lower than 10%. After cultivation in the seed tank for 16 h, the maturity marker of the secondary seed culture was OD562 of 0.7-0.8 × 25, and the secondary seed culture was obtained.
Step 3: (1) Inoculating 10% of the secondary seed culture into the initial fermentation medium in the 500 L fermentation tank, with an initial fermentation temperature of 30°C and pH 6.8. During the fermentation process, continuously adjusting aeration rate and stirring speed to maintain dissolved oxygen at approximately 10%. When the OD562 of the strain in the fermentation broth grew to approximately 40, the pH was adjusted to approximately 7.0, and 1 mM lactose was added at one time (no addition was required for the strain IBCL-1). When the residual sugar in the fermentation broth decreased to 6 wt%, feeding the ammonium sulfate supplementary solution was started to control the osmolality of the fermentation broth at approximately 700 mosm/L. (2) When the residual sugar concentration was lower than 2 wt%, feeding was performed to maintain the residual sugar concentration at approximately 2.0 wt%, and the feeding ratio of the supplementary solution A and the supplementary solution B was controlled at 5/1. Meanwhile, feeding the inorganic salt supplementary solution was continued to control the osmolality of the fermentation broth at approximately 900 mosm/L. The fermentation was terminated when the glucose was completely consumed.

# EP 4 741 505 A1

Table 26 Main Results of Comparative Example 8

| Strai n | Beet Molasses | Cane Molasses | L-Isoleucine wt% | Sugar-To-Acid Conversion Rate % | Byproduct Acid wt% |
|---|---|---|---|---|---|
| IBCI L-253 | 100% | 0 | 6.55 | 37.9 | 0.18 |
| | 0 | 100% | 6.45 | 37.3 | 0.19 |
| | 50% | 50% | 6.35 | 36.7 | 0.2 |
| IBCI L-253k | 100% | 0 | 6.33 | 36.0 | 0.23 |
| | 0 | 100% | 6.28 | 35.7 | 0.25 |
| | 50% | 50% | 6.25 | 35.5 | 0.28 |
| IBCL -1 | 100% | 0 | 6.02 | 34.8 | 0.27 |
| | 0 | 100% | 6.04 | 34.9 | 0.29 |
| | 50% | 50% | 5.95 | 34.4 | 0.3 |

As shown in Comparative Example 8, verification of L-isoleucine fermentation using non-hydrolyzed molasses is performed, and the results are not ideal, failing to achieve the fermentation effect of hydrolyzed molasses. Comparative Example 8 demonstrates that hydrolyzed molasses is more favorable for L-isoleucine fermentation.

**Comparative Example 9: Determination of advantages of new process**

1. The composition of the seed medium:

The primary seed medium: glucose 3 wt%, corn steep liquor powder 1.0 wt%, ammonium sulfate 0.5 wt%, $KH_2PO_4$ 0.1 wt%, $MgSO_4 \cdot 7H_2O$ 0.05 wt%, calcium carbonate 1.0 wt%, pH 6.7-7.2; dispensed at 500 mL/5000 mL; sterilization condition: 121°C/20 min.

Secondary seed tank medium: glucose 5 wt%, corn steep liquor powder 1.0 wt%, ammonium sulfate 0.5 wt%, $KH_2PO_4$ 0.1 wt%, $MgSO_4 \cdot 7H_2O$ 0.05 wt%, pH 6.8-7.2; sterilization condition: 121°C/20 min.

2. The fermentation medium:

The initial fermentation medium in the standard fermentation tank: glucose 12 wt%, corn steep liquor powder 0.8 wt%, ammonium sulfate 0.5 wt%, potassium dihydrogen phosphate 0.1 wt%, magnesium sulfate 0.05 wt%.

Supplementary glucose solution: glucose 40 wt%, potassium dihydrogen phosphate 0.1 wt%, magnesium sulfate 0.05 wt%.

Inorganic salt supplementary solution: ammonium sulfate 10 wt%.

Fermentation waste liquid: a separation waste liquid obtained from fermentation separation, which is rich in ammonium sulfate at about 10-12 wt%.

3. The fermentation tank process:

Step 1: Respectively inoculating one loop of each of the L-isoleucine-producing strains IBCIL-253, IBCIL-253k, and IBCL-1 into the primary seed medium in shake flasks, followed by oscillatory cultivation at 30°C and 90 rpm for 16 h to obtain 500 mL of the shake-flask seed culture. The maturity marker of the primary seed culture was the OD562 of 0.6-0.7 × 25.

Step 2: inoculating the shake-flask seed culture into the 50 L secondary seed tank medium at the ratio of 1% (V/V), followed by fermentation cultivation at 30°C and 90 rpm, with pH 6.8 and a dissolved oxygen level not lower than 10%. After cultivation in the seed tank for 16 h, a maturity criterion of the secondary seed culture was OD562 of 0.7-0.8*25, and the secondary seed culture was obtained.

Step 3: (1) Inoculating the secondary seed culture into the initial fermentation medium in the 500 L fermentation tank at the ratio of 10%, with an initial fermentation temperature of 30°C and pH 6.8. During the fermentation process, aeration rate and the stirring speed were continuously adjusted to control a dissolved oxygen level at about 10%. When OD562 of the strain in the fermentation broth grew to about 40, the pH was adjusted to about 7.0, and 1 mM lactose was added at one time (no addition was required for the strain IBCL-1). When the residual sugar concentration in the fermentation broth decreased to 6 wt%, feeding of the inorganic salt supplementary solution was started to control the fermentation broth osmolality at about 700 mosm/L. (2) When the residual sugar concentration was lower than 1 wt%, feeding of the supplementary glucose solution was performed to control the residual sugar concentration of the fermentation broth at about 1.0 wt%, while feeding of the inorganic salt supplementary solution was continued to control the fermentation broth osmolality at about 900 mosm/L, and the fermentation was terminated when the glucose was completely consumed.

Table 27 Main Results of Comparative Example 9

| Strain | L-Isoleucine wt% | Sugar-to-Acid Conversion Rate % | Byproduct Acid wt% |
|---|---|---|---|
| IBCIL-253 | 66.4 | 38.38 | 0.10 |
| IBCIL-253k | 65.3 | 37.75 | 0.18 |
| IBCL-1 | 61.7 | 36.29 | 0.21 |

As shown in Comparative Example 9, this Comparative Example uses corn steep liquor for L-isoleucine fermentation. By comparison with Example 12, it is observed that using hydrolyzed molasses for L-isoleucine fermentation results in higher acid production and sugar-to-acid conversion rate, with fewer byproduct acids. In addition, molasses contains fewer impurities compared with corn steep liquor, has a lighter color, and is more favorable for subsequent separation and purification.

Hereinafter, the foregoing separation and purification method is further described in detail through a plurality of Examples and Comparative Examples. It should be noted that the reaction conditions, reactants, and amounts of reactants in the Examples are merely for illustration and do not limit the scope of protection of the present disclosure. The Comparative Examples are control groups corresponding to the Examples.

**Example 18: MPN reagent precipitation combined with a composite acid system for inhibiting co-crystallization (purification of L-isoleucine)**

Steps of Example 18:

Preparation of the MPN reagent: polyglutamic acid (10 kDa) and $Fe^{3+}$ were mixed at a molar ratio of 1:2.5 to form a 5% w/v solution at pH 4.5.

Complex precipitation: 1.5% of the MPN reagent was added to a ceramic-filtration filtrate (100 L), stirred for 40 min, and centrifuged (4000 rpm, 15 min) to collect a precipitate.

Salt formation and redissolution: 100 kg of a complex precipitate was taken and mixed with p-toluenesulfonic acid at the molar ratio of 1:1.5, with a solid content of 50%, and dissolved at 60°C.

Hydrogen bond network reconstruction: 1 M hydrochloric acid was added in stages to adjust pH to 1.2, followed by stirring for 30 min.

Gradient crystallization: cooling was performed to 10°C at 10°C/h, seed crystallization was performed for 1 h, and filtration was performed to obtain a crystal cake.

Decolorization: 1.5% malic acid, 0.15% tartaric acid, and 1‰ activated carbon were added for decolorization for 40 min.

Ion exchange: YN201 resin was used for adsorption, and p-toluenesulfonic acid and the resin were regenerated with gradient hydrochloric acid (0.5 M hydrochloric acid for eluting and recovering p-toluenesulfonic acid → 3 M hydrochloric acid for fully regenerating the resin).

Crystallization: a finished product was obtained after the evaporation crystallization.

Main results of Example 18:

Purity: 99.6% (L-isoleucine); impurity L-α-aminobutyric acid: 0.03%; impurity L-norvaline: 0.04%.

Recovery rate of p-toluenesulfonic acid: 85%.

**Comparative Example 10: Single hydrochloric acid system**

Steps of Comparative Example 10: precipitant and composite acid were omitted; only 1 M hydrochloric acid was used for redissolution; and other conditions were the same.

Main results of Comparative Example 10: purity 92.5% (impurity L-α-aminobutyric acid 0.54%; impurity L-norvaline 0.63%).

Conclusion: the composite acid system reduces impurity contents (L-α-aminobutyric acid reduced by 94%, L-norvaline reduced by 94%).

**Example 19: Gradient cooling crystallization to improve purity and yield (L-leucine purification)**

Steps of Example 19:

Salt formation and redissolution: 100 kg crude L-leucine (purity 82%) was taken, p-toluenesulfonic acid was mixed at a molar ratio 1:2, with a solid content of 45%, and dissolved at 55°C.
Hydrogen bond network reconstruction: 1.5 M hydrochloric acid was added to adjust pH to 1.5.
Gradient crystallization: cooling was performed to 15°C at 12°C/h, and seed crystallization was performed for 45 min.
Decolorization: 1% malic acid and 1% tartaric acid were added, and 0.8‰ activated carbon was used for decolorization.
Ion exchange: gradient hydrochloric acid regeneration (0.5 M → 3 M).

Results of Example 19:

Yield: 93%.
Purity: 99.5% (impurity L-norvaline 0.04%).

**Comparative Example 11: Rapid cooling**

Gradient crystallization: a cooling rate of 20°C/h, no seed crystallization step.
Results of Comparative Example 11: purity 97.5% (impurity L-norvaline 0.32%), yield 83%.
Conclusion: gradient cooling combined with the seed crystallization improves yield by 10% and increases purity by 87.5%.

**Example 20: Recycling of the p-toluenesulfonic acid (L-valinepurification)**

Steps of Example 20:

Salt formation and redissolution: 100 kg crude L-valine (purity 80%) was taken, and p-toluenesulfonic acid was mixed at a molar ratio of 1:1.8, with a solid content of 55%.
Ion exchange: YN269 resin was used for adsorption, and 0.5 M hydrochloric acid was used to elute and recover p-toluenesulfonic acid.
Recycling: the recovered p-toluenesulfonic acid was directly used for the next batch.

Results of Example 20:

Recycling rate: 85% (purity ≥95% after five consecutive batches).
Cost comparison: raw material cost is reduced by 40% (in a conventional process, a new precipitant is required for each batch).

**Comparative Example 12: Without recycling**

Steps of Comparative Example 12: new p-toluenesulfonic acid was used in each batch without recovery.
Results of Comparative Example 12: raw material cost increases by 45%, and wastewater COD ≥800 mg/L.
Conclusion: closed-loop regeneration technology reduces raw material cost and decreases wastewater COD to below 200 mg/L.

**Example 21: Optimization of activated carbon dosage (decolorization of mixed amino acids)**

Steps of Example 21:

Salt formation and redissolution: amino acid crude products (leucine + isoleucine + valine) was mixed, p-toluenesulfonic acid was added at a molar ratio of 1:1.2.
Decolorization: 2% malic acid and 1% tartaric acid were added, with 0.5‰ activated carbon for decolorization for 30 min.

Results of Example 21:

Decolorization efficiency: 98% (absorbance $\leq 0.05$).
Activated carbon dosage: 0.5 kg/ton of product.

**Comparative Example 13: Without organic acids**

Steps of Comparative Example 13: malic acid and tartaric acid were omitted, and only activated carbon was used for decolorization (3‰ required).
Results of Comparative Example 13: decolorization efficiency 85% (absorbance $\geq 0.2$), activated carbon dosage 3 kg/ton of product.
Conclusion: organic acids synergistically reduce activated carbon dosage by 83% and improve decolorization efficiency by 13%.

**Example 22: Optimized comprehensive process parameters (L-isoleucine)**

Specific process parameters of Example 22:

Preparation of the MPN reagent: polyglutamic acid (10 kDa) and $Fe^{3+}$ were mixed at a molar ratio of 1:2.5 to form a 5% w/v solution at pH 4.5.
Complex precipitation: 1.5% of the MPN reagent was added to a ceramic-filtration filtrate (100 L), stirred for 40 min, and centrifuged (4000 rpm, 15 min) to collect a precipitate.
Salt formation and redissolution: a molar ratio of 1:1.5, a solid content of 50%, dissolved at 60°C.
Hydrogen bond network reconstruction: 1 M hydrochloric acid was added to adjust pH to 1.2.
Gradient crystallization: cooling was performed to 10°C at 10°C/h, and seed crystallization was performed for 1 h.
Decolorization: 1.5% malic acid and 0.15% tartaric acid were added, with 1‰ activated carbon for decolorization for 40 min.
Ion exchange: YN201 resin was used, 0.5 M hydrochloric acid was used to elute and recover p-toluenesulfonic acid, and 3 M hydrochloric acid was used to regenerate the resin.
Evaporation crystallization: vacuum concentration at 55°C, vacuum degree 0.09 MPa.

Results of Example 22:

Purity: 99.7% (L-isoleucine).
Impurity contents: L-$\alpha$-aminobutyric acid 0.02%, L-norvaline 0.03%.
Total yield: 95%.
Through the comparison between the foregoing Examples and Comparative Examples, the dynamic complex precipitation technology of MPN, the composite acid synergistic system, the gradient crystallization control, and the closed-loop regeneration technology provided in the present disclosure significantly improve the purity and yield of BCAAs, while reducing the dosage of activated carbon and the cost of treatment of three wastes, thereby verifying the efficiency and environmental advantages in industrial production.
The separation and purification method for a fermentation product provided in the Examples of the present disclosure has at least the following advantages:

1. Soluble metal-MPN complex precipitation technology: existing technologies do not use a precipitant or use a single precipitant, whereas in the Examples of the present disclosure, a soluble MPN reagent is added, and dynamic coordination complexation is utilized to selectively precipitate target amino acids, replacing traditional fixed-bed adsorption, thereby simplifying equipment and being adaptable to high-impurity systems.

2. Composite acid impurity-removing system: in the Examples of the present disclosure, a composite acid precipitation synergistic effect (p-toluenesulfonic acid for salt formation, hydrochloric acid for hydrogen bond network destruction, and organic acids for complexing impurities) achieves two beneficial effects: (1) trace-level control of impurity amino acids, reducing each individual impurity amino acid content to below 0.10%; and (2) reduction of activated carbon usage by 50%, reduction of subsequent treatment costs of three wastes, and alleviation of environmental treatment pressure.

3. Recycling of p-toluenesulfonic acid: existing technologies do not involve recycling of key materials and require multiple water or solvent activations or resin regenerations. In the Examples of the present disclosure, recycling of p-toluenesulfonic acid is achieved through gradient elution with hydrochloric acid of different concentrations, thereby further reducing wastewater treatment cost and environmental pressure.

4. Gradient cooling crystallization: existing technologies are mostly evaporation crystallization. In the Examples of the present disclosure, a composite acid system combined with gradient crystallization further avoids the formation of co-crystals, improves the purity of target amino acids in the crystallized cake, controls the residues of impurity amino acids, and simultaneously improves yield.

5. Wide applicability of process flow: existing technologies are mostly targeted at single amino acid products. In the Examples of the present disclosure, without changing equipment, resin, or processing manner, the process flow provided in the present disclosure is applicable to the separation and purification of three BCAAs, with low equipment investment, strong process adaptability, and high continuity, making the process flow suitable for industrial scale-up production.

The separation and purification method for the fermentation product provided by the embodiments of the present disclosure has at least the following advantages.

1. Further reduction of residual impurity amino acids and further improvement of purity of a main-content amino acid: (1) a metal-MPN network serves as a novel adsorbent and precipitant, achieving efficient adsorption and sedimentation of a target amino acid; (2) the p-toluenesulfonic acid forms a stable salt with an amino group of the target amino acid via a sulfonic group, significantly increasing solubility; (3) addition of hydrochloric acid fully protonates amino acids and impurity amino acids and destroys an original hydrogen bond network, and chloride ions ($Cl^-$) in solution provide an ionic shielding effect to inhibit co-crystallization of the byproduct acids;(4) carboxyl groups of organic weak acids form complexes with the metal ions, pigment molecules, and impurity amino acids, further removing contents of impurity amino acids.

2. Reduction of activated carbon dosage: carboxyl groups of organic weak acids form complexes with the metal ions, pigment molecules, and impurity amino acids, improving a decolorization effect of the activated carbon and reducing the activated carbon dosage.

3. Reduction of treatment cost of three wastes: adsorption of p-toluenesulfonic acid by the ion exchange resin in combination with gradient elution with hydrochloric acid achieves recycling of a key material, thereby reducing the post-treatment cost of the precipitant.

4. Strong applicability of equipment and process, flexible product switching, and low investment cost: without changing equipment, resin, or processing manner, the process flow provided in the present disclosure is applicable to separation and purification of three BCAAs, with low equipment investment.

The basic concepts have been described above. Clearly, to those skilled in the art, the above detailed disclosure is merely by way of example and does not constitute a limitation to the present disclosure. Although not explicitly stated herein, those skilled in the art may make various modifications, improvements, and amendments to the present disclosure. Such modifications, improvements, and amendments are suggested in the present disclosure, so they still fall within the spirit and scope of the exemplary embodiments of the present disclosure.

Meanwhile, the present disclosure uses specific words to describe the embodiments of the present disclosure. For example, "one embodiment," "an embodiment," and/or "some embodiments" mean a certain feature, structure, or characteristic related to at least one embodiment of the present disclosure. Therefore, it should be emphasized and noted that the phrases "an embodiment" or "one embodiment" or "an alternative embodiment" mentioned two or more times in different places in the present disclosure do not necessarily refer to the same embodiment. Furthermore, certain features, structures, or characteristics in one or more embodiments of the present disclosure may be appropriately combined.

Furthermore, unless explicitly stated in the claims, the order of processing elements and sequences, the use of numbers and letters, or the use of other names described in the present disclosure are not intended to limit the order of the processes and methods of the present disclosure. Although the above disclosure discusses some inventive embodiments currently considered useful through various examples, it should be understood that such details are for illustrative purposes only, and the appended claims are not limited to the disclosed embodiments. Rather, the claims are intended to cover all modifications and equivalent combinations that conform to the essence and scope of the embodiments of the present disclosure. For example, although the implementation of various components described above may be embodied in a hardware device, it may also be implemented as a software only solution, e.g., an installation on an existing server or mobile device.

Similarly, it should be noted that, in order to simplify the expression of the disclosure of the present disclosure and thereby aid in the understanding of one or more inventive embodiments, the description of the embodiments of the present disclosure above sometimes groups multiple features into one embodiment, drawing, or description thereof. However, this method of disclosure does not mean that the object of the present disclosure requires more features than those mentioned in the claims. Rather, claimed subject matter may lie in less than all features of a single foregoing disclosed embodiment.

In some embodiments, numbers describing the quantity of components or attributes are used. It should be understood that such numbers used in the description of the embodiments are modified by the modifiers "about," "approximately," or "substantially" in some examples. Unless otherwise stated, "about," "approximately," or "substantially" indicates that the stated number allows a variation of $\pm20\%$. Accordingly, in some embodiments, the numerical parameters used in the specification and claims are approximations, which may vary depending on the desired characteristics of individual embodiments. In some embodiments, numerical parameters should consider the indicated significant digits and apply the method of general digit retention. Although the numerical ranges and parameters used to confirm the breadth of their scope in some embodiments of the present disclosure are approximations, in specific embodiments, the setting of such numerical values is as precise as possible within a feasible range.

For each patent, patent application, patent application publication, and other material, such as articles, books, specifications, publications, documents, etc., cited in the present disclosure, their entire contents are hereby incorporated by reference into the present disclosure. Except for application history documents that are inconsistent with or conflict with the content of the present disclosure, and except for documents that limit the broadest scope of the claims of the present disclosure (currently or later appended to the present disclosure). It should be noted that if the description, definition, and/or use of terms in the ancillary materials of the present disclosure are inconsistent with or conflict with those in the present disclosure, the description, definition, and/or use of terms in the present disclosure shall prevail.

Finally, it should be understood that the embodiments described in the present disclosure are only used to illustrate the principles of the embodiments of the present disclosure. Other modifications may also fall within the scope of the present disclosure. Therefore, merely by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A non-coding small RNA (sRNA) from *Corynebacterium glutamicum,* wherein an RNA sequence of the non-coding sRNA has at least 90% sequence identity to a transcription product of a DNA sequence set forth in SEQ ID NO:1.

2. The non-coding sRNA according to claim 1, wherein a DNA sequence encoding the non-coding sRNA is set forth in SEQ ID NO:1.

3. A vector comprising the DNA sequence encoding the non-coding sRNA of claim 2.

4. A recombinant bacterium expressing the DNA sequence encoding the non-coding sRNA of claim 2, wherein a bacterial strain of the recombinant bacterium is *Corynebacterium glutamicum.*

5. The recombinant bacterium according to claim 4, wherein the *Corynebacterium glutamicum* is selected from *Corynebacterium glutamicum* IBBH-15, *Corynebacterium glutamicum* IBCL-1, *Corynebacterium glutamicum* IBCVQ, *Corynebacterium glutamicum* CICC21756, *Corynebacterium glutamicum* ATCC13002, or any combination thereof.

6. A method for constructing a recombinant bacterium, the method comprising:
obtaining a recombinant strain by transferring a recombinant vector comprising a target DNA fragment into a host strain; wherein the target DNA fragment has at least 90% sequence identity to a DNA sequence set forth in SEQ ID NO:1.

7. The method according to claim 6, wherein a DNA sequence of the target DNA fragment is set forth in SEQ ID NO:1.

8. A fermentation method for a recombinant bacterium, the method comprising:

obtaining a seed culture by inoculating at least one recombinant strain into a seed medium for cultivation;
inoculating the seed culture into a fermentation medium for fermentation cultivation; and
in the fermentation cultivation, increasing production of branched-chain amino acids by adjusting at least one of: an added amount of inducer, a dissolved oxygen level, pH, and nutrient supply.

9. The method according to claim 8, wherein the at least one recombinant strain comprises a DNA sequence encoding a non-coding sRNA, and the DNA sequence has at least 90% sequence identity to a DNA sequence set forth in SEQ ID NO:1.

10. The method according to claim 8 or 9, wherein the recombinant bacterium is an L-leucine-producing strain, the L-leucine-producing strain including at least one of *Corynebacterium glutamicum* mutant strains IBBH-15, IBCLQ-257, and IBCLQ-257e.

11. The method according to claim 8 or 9, wherein the seed medium comprises glucose at an initial concentration of 2.5-3.5 wt% and a molasses hydrolysate at an initial concentration of 1-2 wt%, the fermentation medium comprises glucose at an initial concentration of 3.5-4.5 wt% and a molasses hydrolysate at a concentration of 0.5-1.1 wt%, and the molasses hydrolysate is selected from at least one of a beet molasses hydrolysate and a cane molasses hydrolysate.

12. The method according to any one of claims 8-11, wherein a dissolved oxygen level in the fermentation cultivation is 5-10%; the method further comprising:

    in the fermentation cultivation, after a cell OD562 increases to 19-21, increasing pH to 6.85-6.95, and when a residual sugar concentration decreases to 1.5-2.5 wt%, starting to feed a glucose solution having a concentration of 40-50 wt% and a molasses hydrolysate having a concentration of 40-50 wt%, controlling a flow rate ratio of the glucose solution to the molasses hydrolysate to be 5:1-10:1, and controlling a ratio of the molasses hydrolysate to the fermentation medium to be 2.5-3.5 wt%; and
    after the cell OD562 increases to 29-31, increasing the pH to 7.05-7.15, after the cell OD562 increases to 39-41, increasing the pH to 7.15-7.25, and controlling the residual sugar concentration to be 1.5-2.5 wt% during the fermentation process.

13. The method according to claim 8, wherein the recombinant bacterium is an L-isoleucine-producing strain, the L-isoleucine-producing strain comprising at least one of *Corynebacterium glutamicum* mutant strains IBCIL-253, IBCL-1, and IBCIL-253k.

14. The method according to claim 8 or 13, wherein the seed medium comprises glucose at an initial concentration of 4.5-5.5 wt% and a molasses hydrolysate at an initial concentration of 2.5-3.5 wt%, the fermentation medium comprises glucose at an initial concentration of 9.5-10.5 wt% and a molasses hydrolysate at a concentration of 1.5-2.5 wt%, and the molasses hydrolysate is selected from at least one of a beet molasses hydrolysate and a cane molasses hydrolysate.

15. The method according to any one of claims 8, 13, and 14, wherein a dissolved oxygen level in the fermentation cultivation is 8-12%; the method further comprising:

    in the fermentation cultivation, after a cell OD562 increases to 39-41, increasing pH to 6.85-6.95;
    when a residual sugar concentration decreases to 5.5-6.5 wt%, starting to feed an inorganic salt supplementary solution and controlling a fermentation broth osmolality to be 690-710 mosm/L; and
    when a residual sugar concentration decreases to 1.5-2.5 wt%, starting to feed a supplementary solution A and a supplementary solution B, the supplementary solution A comprising a glucose solution at a concentration of 40-50 wt%, a potassium dihydrogen phosphate solution at a concentration of 0.1 wt%, and a magnesium sulfate solution at a concentration of 0.05 wt%, the supplementary solution B comprising a molasses hydrolysate at a concentration of 40-50 wt%, controlling a flow rate ratio of the supplementary solution A to the supplementary solution B to be 4:1-7:1, controlling a ratio of the molasses hydrolysate to the fermentation medium to be 3.5-4.5 wt%, and continuing to feed the inorganic salt supplementary solution to control the fermentation broth osmolality to be 890-910 mosm/L.

16. A separation and purification method for a fermentation product, the method comprising:

    redissolving the fermentation product using p-toluenesulfonic acid, wherein a molar ratio of the fermentation product to the p-toluenesulfonic acid is 1:1-1:2;
    performing a hydrogen bond network reconstruction on the redissolved fermentation product using 0.5-1.5 M inorganic acid;
    decolorizing the fermentation product after cooling crystallization for 30-50 min using 1-2% malic acid, 0.1-0.2% tartaric acid, and 0.5-1.5‰ activated carbon; and
    purifying the decolorized fermentation product by ion exchange resin and evaporation crystallization to obtain a purified fermentation product.

17. The method according to claim 16, wherein after ion exchange resin and before evaporation crystallization, the method further comprises:
eluting and recovering p-toluenesulfonic acid using 0.3-0.8 M hydrochloric acid, and regenerating the resin using 2.5-3.5 M hydrochloric acid.

18. The method according to claim 16 or 17, wherein after the hydrogen bond network reconstruction and before decolorization, the method further comprises:
cooling the fermentation product after the hydrogen bond network reconstruction to 9-11°C at a cooling rate of 8-12°C/h, and performing seed crystallization for 0.5-1.5 h.

19. The method according to any one of claims 16-18, wherein before redissolution, the method further comprises:
performing complex precipitation on a fermentation broth containing the fermentation product using a composition of polypeptides and metal ions to precipitate the fermentation product.

20. The method according to any one of claims 16-19, wherein the fermentation product is a *Corynebacterium glutamicum* fermentation product.

21. The method according to claim 20, wherein the *Corynebacterium glutamicum* comprises a DNA sequence set forth in SEQ ID NO:1.

**Fig 1**

EP 4 741 505 A1

## INTERNATIONAL SEARCH REPORT

| International application No. |
|---|
| **PCT/CN2025/084237** |

| **A.** | **CLASSIFICATION OF SUBJECT MATTER** |
|---|---|

C12N 15/113(2010.01)i; C12P13/00(2006.01)i; C12R1/225(2006.01)i; C07C229/22(2006.01)i; C12R1/19(2006.01)i; C12P21/02(2006.01)i; C12N15/74(2006.01)i

According to International Patent Classification (IPC) or to both national classification and IPC

| **B.** | **FIELDS SEARCHED** |
|---|---|

Minimum documentation searched (classification system followed by classification symbols)

IPC:C12N,C12P,C12R,C07C

Documentation searched other than minimum documentation to the extent that such documents are included in the fields searched

Electronic data base consulted during the international search (name of data base and, where practicable, search terms used)

CNABS, DWPI, CNTXT, WOTXT, JPTXT, USTXT, EPTXT, 万方, WANFANG, CNKI, PubMed, GenBank, ISI web of knowledge, 中国专利序列数据库, China Patents Biological Sequence Database: 谷氨酸棒杆菌, 非编码Srna, SEQ ID NO.1, 转录产物, L-亮氨酸, L-异亮氨酸, L-缬氨酸, 支链氨基酸, 糖酸转化率, 副产物, 糖蜜, 碳氮源配比, 补料, Corynebacterium glutamicum, non-encoded Srna, SEQ ID NO. 1, transcript, L-leucine, L-isoleucine, L-valine, branched chain amino acids, sugar acid conversion, by-products, molasses, carbon nitrogen mix, feeds

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 118086306 A (INNOBIO CORP., LTD. et al.) 28 May 2024 (2024-05-28) claims 1-10, and description, paragraphs 27-77 | 1-21 |
| X | CN 108841758 A (INNOBIO CORP., LTD.) 20 November 2018 (2018-11-20) claims 1-5, and description, paragraphs 10-28 and 38 | 8, 10-12 |
| X | CN 116121135 A (INNOBIO CORP., LTD. et al.) 16 May 2023 (2023-05-16) claims 1-10, and description, paragraphs 14-37 | 8, 13-15 |
| A | CN 106434672 A (TIANJIN UNIVERSITY) 22 February 2017 (2017-02-22) claims 1-4, and sequence 1 | 1-21 |
| A | CN 104531795 A (BEIJING GLECKES BIO-ENGINEERING TECHNOLOGY CO., LTD.) 22 April 2015 (2015-04-22) claims 1-4 | 1-21 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **31 May 2025** | **10 June 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2025/084237**

### C.   DOCUMENTS CONSIDERED TO BE RELEVANT

| Category* | Citation of document, with indication, where appropriate, of the relevant passages | Relevant to claim No. |
|---|---|---|
| A | CN 108299220 A (HENAN JULONG BIOLOGICAL ENGINEERING CO., LTD.) 20 July 2018 (2018-07-20) <br> claims 1-10 | 1-21 |
| A | CN 113817627 A (INNOBIO CORP., LTD.) 21 December 2021 (2021-12-21) <br> claims 1-10 | 1-21 |
| A | WO 2005098895 A2 (CALIFORNIA INSTITUTE OF TECHNOLOGY) 20 October 2005 (2005-10-20) <br> claims 1-81 | 1-21 |

Form PCT/ISA/210 (second sheet) (July 2022)

**INTERNATIONAL SEARCH REPORT**
Information on patent family members

International application No.

**PCT/CN2025/084237**

| Patent document cited in search report | | | Publication date (day/month/year) | Patent family member(s) | | | Publication date (day/month/year) |
|---|---|---|---|---|---|---|---|
| CN | 118086306 | A | 28 May 2024 | None | | | |
| CN | 108841758 | A | 20 November 2018 | None | | | |
| CN | 116121135 | A | 16 May 2023 | None | | | |
| CN | 106434672 | A | 22 February 2017 | None | | | |
| CN | 104531795 | A | 22 April 2015 | None | | | |
| CN | 108299220 | A | 20 July 2018 | None | | | |
| CN | 113817627 | A | 21 December 2021 | None | | | |
| WO | 2005098895 | A2 | 20 October 2005 | US | 2008017796 | A1 | 24 January 2008 |
| | | | | US | 7442931 | B2 | 28 October 2008 |
| | | | | US | 2005253069 | A1 | 17 November 2005 |
| | | | | US | 7154091 | B2 | 26 December 2006 |
| | | | | EP | 1735811 | A2 | 27 December 2006 |
| | | | | EP | 1735811 | A4 | 19 August 2009 |
| | | | | EP | 1735811 | B1 | 09 September 2015 |
| | | | | JP | 2011108657 | A | 02 June 2011 |
| | | | | JP | 5437284 | B2 | 12 March 2014 |
| | | | | US | 2009236521 | A1 | 24 September 2009 |
| | | | | US | 7915583 | B2 | 29 March 2011 |
| | | | | JP | 2013243157 | A | 05 December 2013 |
| | | | | JP | 5684869 | B2 | 18 March 2015 |
| | | | | JP | 2007531876 | A | 08 November 2007 |
| | | | | WO | 2005098895 | A9 | 16 March 2006 |
| | | | | WO | 2005098895 | A3 | 07 December 2006 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410335441